(19) Europäisches Patentamt
European Patent Office
Office européen des brevets

(11) **EP 3 244 911 B1**

(12) **EUROPEAN PATENT SPECIFICATION**

(45) Date of publication and mention
of the grant of the patent:
**23.10.2019 Bulletin 2019/43**

(21) Application number: **16700794.7**

(22) Date of filing: **08.01.2016**

(51) Int Cl.:
*A61K 38/16* (2006.01)     *A61K 38/18* (2006.01)
*A61K 38/29* (2006.01)     *A61K 39/395* (2006.01)
*A61K 31/663* (2006.01)     *A61P 15/08* (2006.01)
*A61K 38/13* (2006.01)

(86) International application number:
**PCT/DK2016/050005**

(87) International publication number:
**WO 2016/112916 (21.07.2016 Gazette 2016/29)**

(54) **A METHOD FOR DETERMINING A LIKELY EFFECT OF A TREATMENT TO IMPROVE MALE INFERTILITY**

EINE METODE ZUR BESTIMMUNG EINES WAHRSCHEINLICHEN EFFEKTS EINER BEHANDLUNG ZUR VERBESSERUNG MÄNNLICHER UNFRUCHTBARKEIT

METODE POUR DETERMINER UN EFFET PROBABLE POUR LE TRAITEMENT POUR AMELIORER L'INFERTILITE MUSCULINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.01.2015 DK 201570010**

(43) Date of publication of application:
**22.11.2017 Bulletin 2017/47**

(73) Proprietor: **Rigshospitalet, Copenhagen University Hospital**
**2100 Copenhagen Ø (DK)**

(72) Inventor: **JENSEN, Martin Blomberg**
**2100 Copenhagen Ø (DK)**

(74) Representative: **Plougmann Vingtoft a/s**
**Strandvejen 70**
**2900 Hellerup (DK)**

(56) References cited:
**WO-A1-2007/020042     WO-A1-2015/018421**
**WO-A1-2015/018421     WO-A1-2015/018421**

• **U.S. National Library Of Medicine: "Archive History for NCT02422108", , 13 May 2015 (2015-05-13), XP055512614, Retrieved from the Internet: URL:https://clinicaltrials.gov/ct2/history /NCT02422108?V_5=View#StudyPageTop [retrieved on 2018-10-05]**
• **NORIAN J M ET AL: "Low serum osteoprotegerin levels in premenopausal infertile women with low bone density: an association of relevance?", FERTILITY AND STERILITY, ELSEVIER SCIENCE INC, NEW YORK, NY, USA, vol. 91, no. 2, 1 February 2009 (2009-02-01), pages 542-548, XP025966899, ISSN: 0015-0282, DOI: 10.1016/J.FERTNSTERT.2007.11.089 [retrieved on 2009-02-01]**
• **M. COHEN-LAHAV ET AL: "Vitamin D decreases NF B activity by increasing I B levels", NEPHROLOGY DIALYSIS TRANSPLANTATION., vol. 21, no. 4, 1 April 2006 (2006-04-01), pages 889-897, XP055255529, GB ISSN: 0931-0509, DOI: 10.1093/ndt/gfi254**
• **M. BLOMBERG JENSEN ET AL: "Vitamin D is positively associated with sperm motility and increases intracellular calcium in human spermatozoa", HUMAN REPRODUCTION, vol. 26, no. 6, 1 June 2011 (2011-06-01), pages 1307-1317, XP055255553, GB ISSN: 0268-1161, DOI: 10.1093/humrep/der059**

**(Cont. next page)**

- MARTIN BLOMBERG JENSEN: "Vitamin D and male reproduction", NATURE REVIEWS. ENDOCRINOLOGY, vol. 10, no. 3, 14 January 2014 (2014-01-14), pages 175-186, XP055255554, US ISSN: 1759-5029, DOI: 10.1038/nrendo.2013.262
- U. AHSAN ET AL: "Role of selenium in male reproduction-A review", ANIMAL REPRODUCTION SCIENCE., vol. 146, no. 1-2, 1 April 2014 (2014-04-01), pages 55-62, XP055255545, NL ISSN: 0378-4320, DOI: 10.1016/j.anireprosci.2014.01.009
- SONIA SHALINI ET AL: "Alterations in selenium status influences reproductive potential of male mice by modulation of transcription factor NF[kappa]B", BIOMETALS, KLUWER ACADEMIC PUBLISHERS, BO, vol. 20, no. 1, 7 June 2006 (2006-06-07), pages 49-59, XP019464033, ISSN: 1572-8773
- FONG-FU CHOU ET AL: "Spermatogenesis after parathyroidectomy in patients with symptomatic secondary hyperparathyroidism", JOURNAL OF THE AMERICAN COLLEGE OF SURGEONS., vol. 196, no. 6, 1 June 2003 (2003-06-01), pages 854-858, XP055255550, US ISSN: 1072-7515, DOI: 10.1016/S1072-7515(03)00129-7
- Q. FU ET AL: "Parathyroid Hormone Stimulates Receptor Activator of NF B Ligand and Inhibits Osteoprotegerin Expression via Protein Kinase A Activation of cAMP-response Element-binding Protein", JOURNAL OF BIOLOGICAL CHEMISTRY, vol. 277, no. 50, 2 October 2002 (2002-10-02), pages 48868-48875, XP055255667, US ISSN: 0021-9258, DOI: 10.1074/jbc.M208494200
- VIRVE PENTIKÄINEN: "Nuclear Factor-KappaB Activation in Human testicular Apoptosis", AMERICAN JOURNAL OF PATHOLOGY, vol. 160, no. 1, 1 January 2002 (2002-01-01), pages 205-218, XP055255677,
- HUANG JOHN C ET AL: "PTH differentially regulates expression of RANKL and OPG.", February 2004 (2004-02), JOURNAL OF BONE AND MINERAL RESEARCH : THE OFFICIAL JOURNAL OF THE AMERICAN SOCIETY FOR BONE AND MINERAL RESEARCH FEB 2004, VOL. 19, NR. 2, PAGE(S) 235 - 244, XP009188922, ISSN: 0884-0431 abstract
- TIAN J ET AL: "Parathyroid hormone-parathyroid hormone related protein receptor messenger RNA is present in many tissues besides the kidney.", 1993, AMERICAN JOURNAL OF NEPHROLOGY 1993, VOL. 13, NR. 3, PAGE(S) 210 - 213, XP009188920, ISSN: 0250-8095 abstract
- U.S. National Library Of Medicine: "Archive History for NCT02422108", , 13 May 2015 (2015-05-13), XP055512614, Retrieved from the Internet: URL:https://clinicaltrials.gov/ct2/history /NCT02422108?V_5=View#StudyPageTop [retrieved on 2018-10-05]
- NORIAN J M ET AL: "Low serum osteoprotegerin levels in premenopausal infertile women with low bone density: an association of relevance?", FERTILITY AND STERILITY, ELSEVIER SCIENCE INC, NEW YORK, NY, USA, vol. 91, no. 2, 1 February 2009 (2009-02-01), pages 542-548, XP025966899, ISSN: 0015-0282, DOI: 10.1016/J.FERTNSTERT.2007.11.089 [retrieved on 2009-02-01]

**Description**

**Technical field of the invention**

The present invention relates to a method for determining a likely effect of a treatment to improve male fertility.

**Background of the invention**

The receptor activator of NF-κB ligand (RANKL) system is considered important for bone homeostasis and comprises three important factors. RANKL is a transmembrane ligand that binds to a specific receptor (receptor activator of NF-κB (RANK)) on a neighbour cell that subsequently activates NFKB and regulates cellular activation through regulation of cell cycle i.e proliferation, differentiation and apoptosis. OPG is an endogenous secreted protein that binds RANKL and inhibits its signalling.This system is regulated and influenced by various factors. Some paracrine signals influence osteoblast and osteoclast interaction and thereby RANKL signalling such as Sclerostin, Cathepsin K, Wnt signalling, Dickkopf-related protein 1 (DKK1), WNT, Kremen, Frizzled and B-catenin. In addition several endocrine factors such as PTH, calcitonin, estradiol, testosterone are regulators of the RANKL system and the dynamic cellular interaction between the bone cells.

*RANK/RANKL*

RANK/RANKL triggers a network of TRAF-mediated kinase cascades that promote osteoclast differentiation. RANKL is expressed on osteoblast cells and its receptor, Rank, on pre- osteoclastic cells. RANKL expression is stimulated by a number of factors, such as IL-1, IL-6, IL-11 , IL-17, TNF- α, vitamin D, $Ca^{2+}$, parathyroid, glucocorticoids, prostaglandin E2, and immunosuppressive drugs, and is downregulated by TGF-α. The RANK/RANKL interaction induces differentiation and formation of multinucleated mature osteoclasts, causing bone resorption. The third protein, osteoprotegerin (OPG), is produced by fibroblasts in the skeleton and is known to exert an inhibitory effect on the pre-osteoclastic differentiation process. By binding to RANKL also known as osteoprotegerin binding protein (OPGbp), OPG inhibits the RANK/RANKL interaction and subsequent osteoclastogenesis. OPG is thus a very efficient anti-resorptive agent. It also serves as a decoy receptor for the tumour necrosis factor-related apoptosis-inducing ligand (TRAIL) and increases cell survival by blocking the apoptotic effects of this ligand and RANKL. The fact that the overexpression of OPG in mice results in severe osteopetrosis and that OPG-null mice are osteoporotic is a testimony to the physiological importance of OPG. Likewise, the lack of RANK or RANKL induces osteopetrosis in mice.
Thus, the RANK/RANKL system is vital for activation of the bone resorping cells (osteoclasts). In bone the synthesizing cells (osteblasts) express RANKL that signals to RANK on the immature osteoclasts. This induces proliferation and activation of the cells they start to proliferate and resorp bone. OPG is produced by somatic cells in the bone and this production is regulated by sex hormones, TGF-B and various other substances. Today a human made recombinant antibody against RANKL, Denosumab is used to treat osteoporosis as it inhibits RANKL signalling and thus causes less bone resorption in humans. RANKL signalling has only two other known additional functions in healthy humans where it is involved in lactation in the breast and immune cells. Recently, use of RANK/RANKL antagonists for treating neuromuscular disorders, genetic myopathies and/or non-genetic myopathies and/or for regulating skeletal and cardiac muscle disuse, diseases and aging have also been disclosed (WO 2013/067639 A1).

*Sclerostin*

Sclerostin is a protein that in humans is encoded by the SOST gene. It is a secreted glycoprotein with sequence similarity to the family of bone morphogenetic protein (BMP). Sclerostin is produced by osteocytes some chondrocytes and has anti-anabolic effects on bone formation. Sclerostin binds to LRP5/6 receptors and inhibits the Wnt signaling pathway, which leads to decreased bone formation. Although the underlying mechanisms are unclear, it is believed that the antagonism of BMP-induced bone formation by sclerostin is mediated by Wnt signaling. Sclerostin antagonists have been developed in several companies for instance the antibody Romosozumab, which is currently being validated to treat osteoporosis. The osteocyte product sclerostin is an important paracrine regulator of bone mass and exogenous treatment of osteocytes with sclerostin increases RANKL-mediated osteoclast activity, which indicates that at least part of the anabolic bone activity of Sclerostin inhibition may be due to inhibition of the RANKL system.

*Cathepsin k*

The protein encoded by this gene is a lysosomal protease involved in bone remodeling and resorption, and, is expressed predominantly in osteoclasts. Cathepsin K is defined by its high specificity for kinins, that is involved in bone resorption. The enzyme's ability to catabolize elastin and collagen allow it to break down bone and cartilage. Cathepsin K inhibitors, such as odanacatib and VEL-0230, appears to be very potent for the treatment of osteoporosis. Cathepsin K expression is stimulated by cytokines that are released after tissue injury. Besides Cathepsin K several other factors that also directly modulate osteoclastic bone resorption, including cytokines (RANK ligand, tumor necrosis factor-alpha and interferon gamma), hormones (retinoic acid and estrogen) and nuclear transcriptional factors (c-jun and Mitf) also regulate cathepsin K gene expression. It has been reported that cathepsin K is expressed in mesenchymal stem cells, osteoblasts, osteocytes, and bone marrow stromal cells (BMSCs) in humans, albeit at much lower levels than in osteoclasts. RANKL, NFAT, Mitf, and various components enhance osteoclast formation and bone resorption, whereas IFN-gamma, calcitonin, estradiol, and calcium inhibit it. These agents appear to act correspondingly to alter cathepsin K mRNA and protein expression in order to stimulate and suppress the osteoclast's resorbing potential. RANKL signaling via the calcineurin-calcium-NFAT signaling cascade plays a significant role in the regulation of cathepsin K expression.

## NFAT

Nuclear factor of activated T cells (NFAT) activity especially NFATc1 is crucial for the differentiation of bone-resorbing osteoclasts. Inhibition induces the reduction of bone mass despite a blockade of osteoclast differentiation. This reduction is caused by severe impairment of bone formation, suggesting that NFAT transcription factors also have an important role in the transcriptional program of osteoblasts. In fact, bone formation is inhibited in Nfatc1- and Nfatc2-deficient cells. Overexpression of NFATc1 stimulates Osterix8-dependent activation of the Col1a1 (encoding type I collagen) promoter, but not Runx2-dependent activation of osteocalcin. NFAT and Osterix form a complex that binds to DNA, and this interaction is important for the transcriptional activity of Osterix. Thus, NFAT and Osterix cooperatively control osteoblastic bone formation Important for bone regeneration in osteopenic disease (Nature Medicine 11, 880 - 885 (2005) doi:10.1038/nm1270). RANKL, NFAT and various components enhance osteoclast formation and bone resorption and alter cathepsin K mRNA and protein expression in order to stimulate and suppress the osteoclast's resorbing potential.

## Dickkopf-related proteins (DKK's) especially 1 (DKK1), WNT, Kremen, Frizzled and B-catenin

DKK's are proteins and number 1 is in humans encoded by the DKK1 gene and is a member of the dickkopf family. It is a secreted protein involved in embryonic development through its inhibition of the WNT signaling pathway. DKK1 also interacts with LRP6 and is upregulated by androgens.

The Wnt/β-catenin pathway is a major signaling cascade in bone biology. DKK1 plays a role in regulating bone development and remodeling, while Serum DKK1 expression was correlated with lumbar and femoral t- and z-scores in adults. Serum DKK1 concentration were higher in patients with osteoporosis than controls. DKK1, a powerful antagonist of canonical Wnt signaling dependent on Frizzled, Kremen etc, may have a role to play for disorders of bone mass. DKK1 antibodies are now being tested for their efficacy in treating bone disease for instance DKN-01 and/or BHQ880. Other classical WNt inhibitors are sFRP, Dkk, WIF,Wise/SOST, Cerberus, IGFBP, Shisa, Waif1, APCDD1, and Tiki1. On the other hand the Wnt activators, R-spondin and Norrin, promote Wnt signaling by binding to Wnt receptors or releasing a Wnt-inhibitory step.

Decreased semen quality is a major factor of male infertility. Semen quality is a measure of the ability of the semen to accomplish fertilization. Evaluation of male fertility potential is today basically conducted through semen analysis. A semen analysis evaluates certain characteristics of a male's semen and the spermatozoa contained in the semen. The most common variables measured to evaluate sperm quality are: sperm count, motility and morphology.

However, there is no known treatment for men with no sperm in the ejaculate or a drug that can increase sperm number today.

## Summary of the invention

We have surprisingly found that most of the bone factors that are closely interconnected in the skeleton also are present in the male gonad. This clearly indicate that bone cells share many similarities with germ cells and that more scientific focus should be moved in this direction. This imply that for instance RANKL and Sclerostin may induce a marked response in the gonad directly because the ligands and their receptors are there. It is thus considered likely that many other bone factors are expressed in the gonads may have an unique effect on germ cell number there.

## RANKL

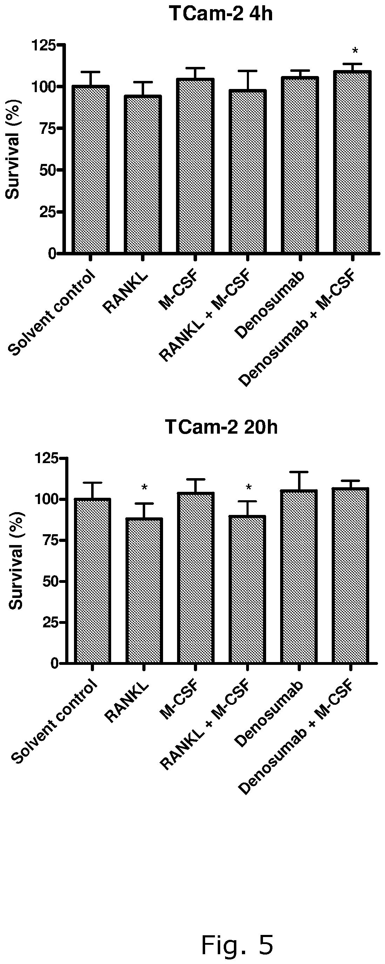

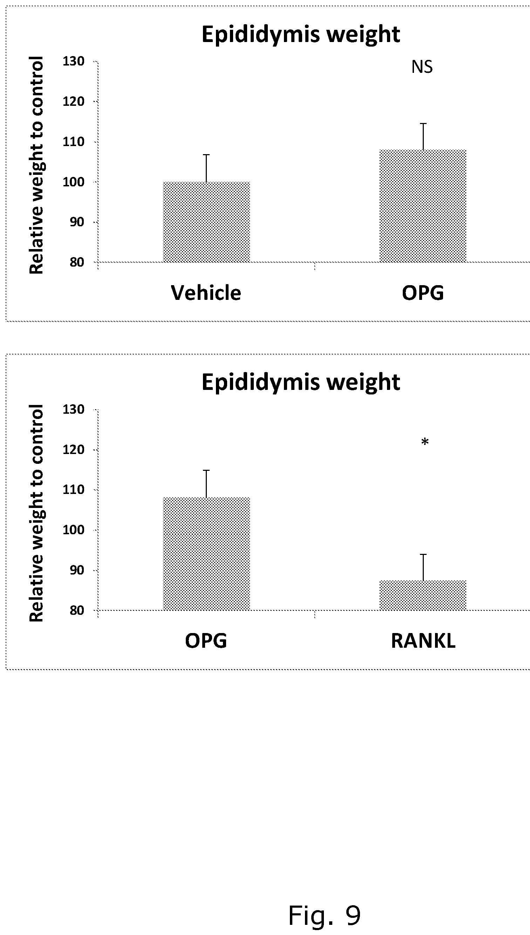

In biopsies with spermatogenic failure or severe testicular dysgeneis with carcinoma *in situ,* it seems as if the production of OPG in the peritubular cells vanishes. This indicates that it is possible to treat humans with an antagonist or inhibitor of RANKL, such as antagonistic anti-RANKL antibodies, which may influence proliferation and apoptosis in the germ cells. Therefore, antagonistic anti-RANKL antibodies, such as Denosumab, may have a new indication as the treatment of men with no spermatozoa but presence of germ cells in the testis. The counterpart (RANKL) can be used to obtain the opposite effect male anticonception. As indicated by the effects shown in figure 5, where RANKL reduces germ cell proliferation in opposition to Denusomab in the *in vitro* model and in figure 9, where RANKL treatment in vivo reduces epididymal weigth unlike the significant increase following Denusomab treatment.The present inventor has shown that RANKL, RANK and OPG are expressed on both RNA and protein level in the human and mouse testis. The Sertoli cells express RANKL, while the germ cells express RANK and the peritubular cells express OPG. Normally, RANKL activates NFKB and activation of this pathway in the male gonad determines whether the testicular cells proliferate or undergo apoptosis in the testis. Therefore, this pathway appears to be a novel regulator of germ cell proliferation. Importantly, this pathway is heavily influenced by several paracrine factors in the skeleton. Here, we show for the first time that most of these factors also are expressed in the testis, which indicates that these factors also modulate RANKL signalling and Sertoli-germ cell interaction. We suggest that these factors also influence germ cell proliferation. Another possibility is to use drugs developed to inhibit for instance Sclerostin, DKK1, Cathepsin K, NFAT, TRAIL and Wnt signalling. Such drugs are clinically being tested now in our laboratory. Besides the direct effect on germ-Sertoli cell interaction they may also influence other growth signals important for bone and germ cell proliferation for instance bone morphogenetic proteins (BMP) such as BMP2, BMP4, BMP6 and BMP7 in a dose-dependent manner.

*Sclerostin (SOST)*

The current data indicate that Sclerostin (SOST) antagonists (e.g. the antibody Romosozumab) can also be used to regulate germ cell proliferation and therefore be used to treat male infertility or as an contraceptive depending on the use of antagonist or agonist for instance sclerostin versus anti-sclerostin.

*Cathepsin k*

The current data indicate that Cathepsin K inhibitors (such as Odanacatib, OST-4077 and VEL-0230) can also be used to regulate germ cell proliferation and therefore be used to treat male infertility or as an contraceptive depending on the use of antagonist or agonist for instance Cathepsin K versus anti-Cathepsin K.

*NFAT*

The current data indicate that Nuclear factor of activated T cells (NFAT) inhibitors (such as $C_{75}H_{118}N_{20}O_{22}S$ or cyclosporine A) can also be used to regulate germ cell proliferation and therefore be used to treat male infertility or as an contraceptive depending on the use of antagonist or agonist for instance NFAT versus anti-NFAT.

*Dickkoof-related protein 1 (DKK1), WNT, Kremen, Frizzled and B-catenin.*

The current data indicate that DKK1, DKK2, WNT, Kremen, Frizzled inhibitors among others can also be used to regulate germ cell proliferation and therefore be used to treat male infertility or as an contraceptive depending on the use of antagonist or agonist for instance DKK1 versus anti-DKK1.

*Parathyroid hormone (PTH) and PTH related peptid (PTHrP)*

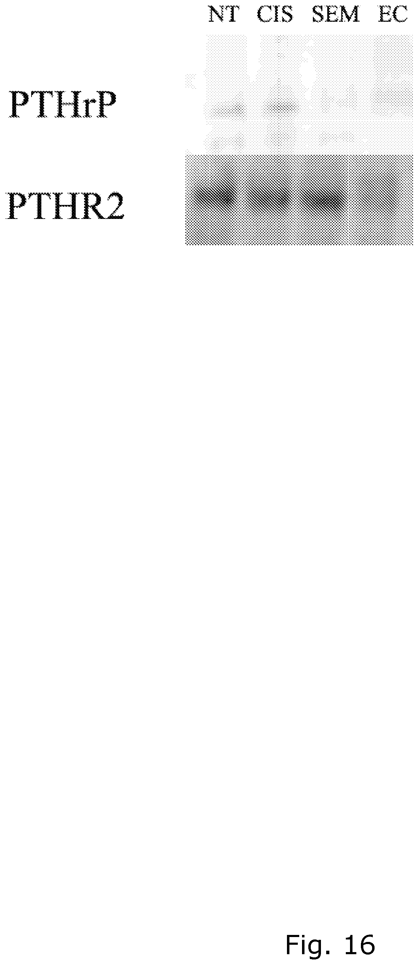

As shown in Figure 16, WB confirms the presence of PTH related peptid (PTHrP) and the PTH receptor in normal testis and CIS indicating that this regulator of RANKL signalling also may play a role for male reproductive health. It is known that PTH and PTHrP is a powerful regulator of bone cell interaction and RANKL signaling. Local PTHrP production may therefore be important for regulating germ cell proliferation.

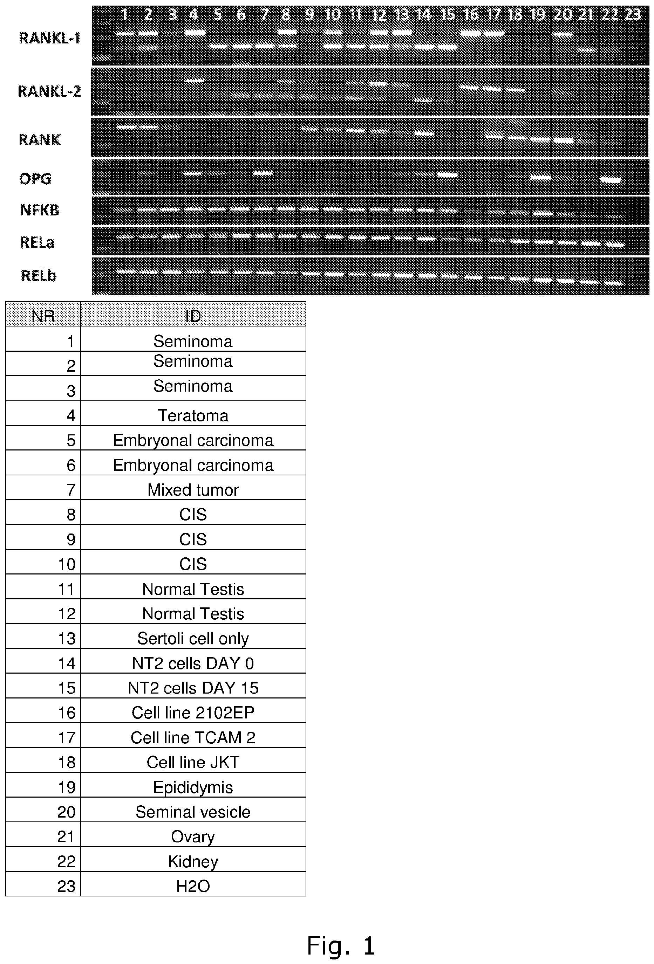

As can be seen on Figure 1, one isoform of RANKL, RANK and OPG are also expressed in the OVARY. This indicates that the RANKL system also is important for female reproduction and we suggest that modulation of the RANKL system also may be of importance for female reproductive function. By using inhibition with OPG or denusomab and alternatively RANKL to either stimulate reproductive function or used as a female contraceptive.

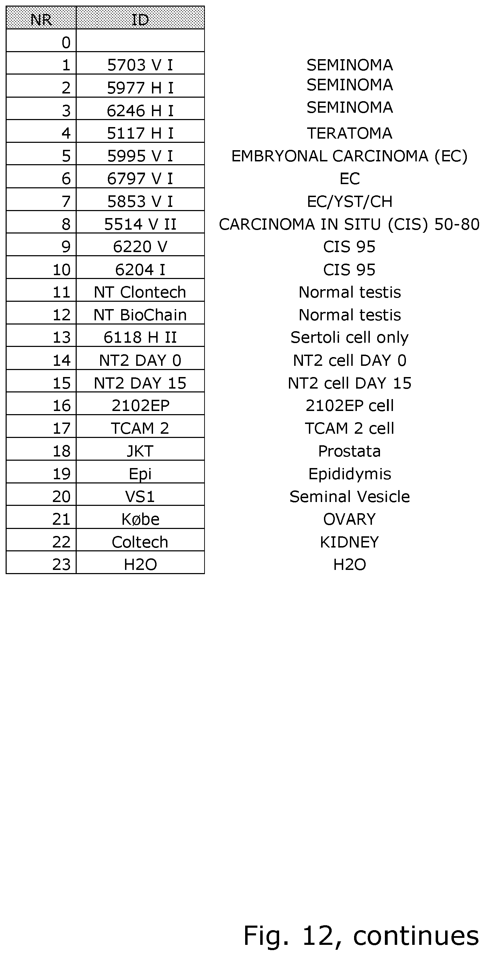

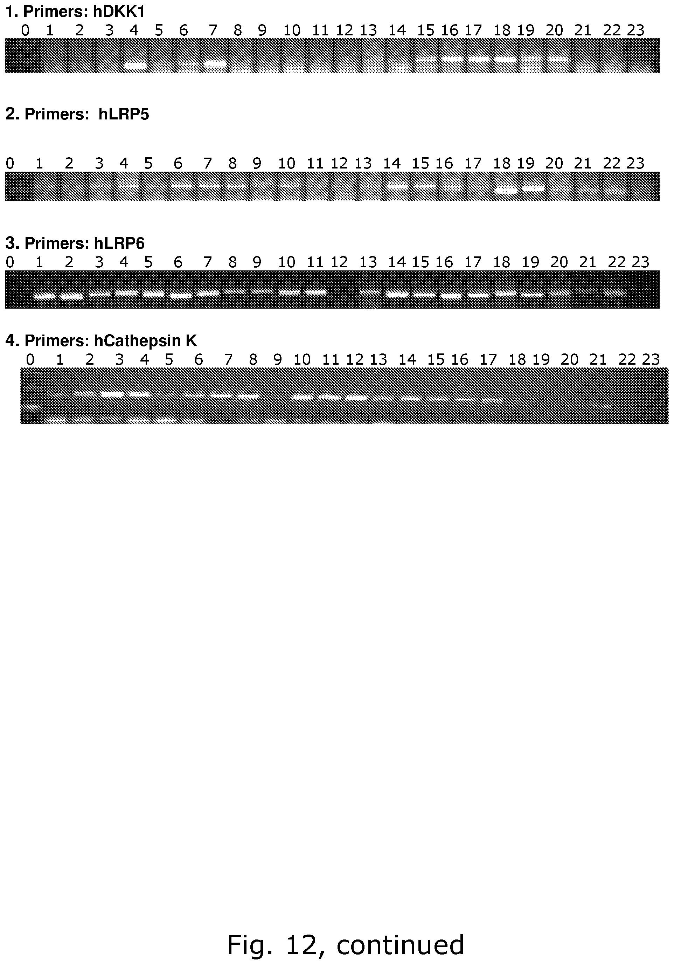

This indicates that all the mentioned factors DKK1, Cathepsin K, Sclerostin etc also has the potential to influence female reproduction and stimulators and inhibitors of these pathways can be used to improve female fertility or as an contraceptive. We show here in Figure 12 that the receptors LRP5 and LRP6 for these compounds are expressed in the ovary and the ligands for instance cathepsin k is also expressed.

*Fertility*

Decreased semen quality is a major factor of male infertility. Semen quality is a measure of the ability of the semen to accomplish fertilization. Evaluation of male fertility potential is today basically conducted through semen analysis. A semen analysis evaluates certain characteristics of a male's semen and the spermatozoa contained in the semen. The characteristics measured by the current tests for semen analysis are only some of the clinical important factors for semen quality. The most common variables measured to evaluate sperm quality are: sperm count, motility and morphology. Other variables are volume, fructose level and pH.

In particular it would be of great benefit to have a test, which could predict whether the person should continue at home the natural way or with the mild insemination or if the couple should proceed to the more invasive IVF or ICSI. Thus, developing a marker that can guide the doctor in this choice would be a clear advantage.

Thus, in an aspect the invention relates to a method for determining a likely effect of a treatment to improve male fertility, the method comprising

- determining the level of OPG in a blood serum sample, which has been obtained from a male subject;

- comparing said determined OPG level to a reference level; and

- determining that said subject will

   ○ likely not benefit from treatment with an antagonist or inhibitor of RANKL, if said level is lower than said reference level, or

   ○ likely benefit from treatment with an antagonist or inhibitor of RANKL, if said level is equal to higher than said reference level;

wherein said reference level is above 3 pmol/l OPG in blood serum.

Thus, we suggest that you can use serum markers of the corresponding bone factors (agonists) or their putative circulating antagonist to predict who will benefit from treatment with antibodies or inhibitors of RANKL, Sclerostin, Cathepsin RANKL and/or DKK1 (preferably OPG, see example 8). Serum levels of these factors may be of assistance in deciding which patients that will benefit from a compound, which is an antagonist or inhibitor of e.g. Schlerostin Cathepsin K, RANKL or DKK1 binding to their respective receptors, for use in stimulation of (germ) cell proliferation in the testes, and/or for use in treatment and/or prevention of male infertility in mammals, and/or for use in inducing or improving male fertility in mammals.

To use serum levels of e.g. OPG, Sclerostin, RANKL, DKK1, Cathepsin K to select the men suitable for treatment with an appropriate agent. For instance men with low serum OPG and/or high serum RANKL may theoretically be more susceptible to Denusomab treatment and likewise for men with high serum levels of DKK1, Sclerostin, Cathepsin before deciding to initiate antibody or inhibition of one of these factors (see e.g. example 7).

**Brief description of the figures**

Figure 1

Figure 1 shows RT-PCR of selected transcripts from human specimens marked from 1-23. Two different primersets detected two different isoforms of RANKL in normal and pathological testicular specimens. RANK and OPG are also expressed in normal and pathological testicular specimens, and OPG expression vanishes in CIS and seminoma compared with normal testis. Interestingly, only one RANKL isoform is expressed in Sertoli cell and this isoform is not expressed in the ovary indicating a sex-specific RANKL-isoform. This isoform is not expressed in NTera2 cells, while it is expressed in TCAM2 cells. RANK is absent or weakly expressed in non-seminomas while NFKB and RELa+b are expressed in all specimens.

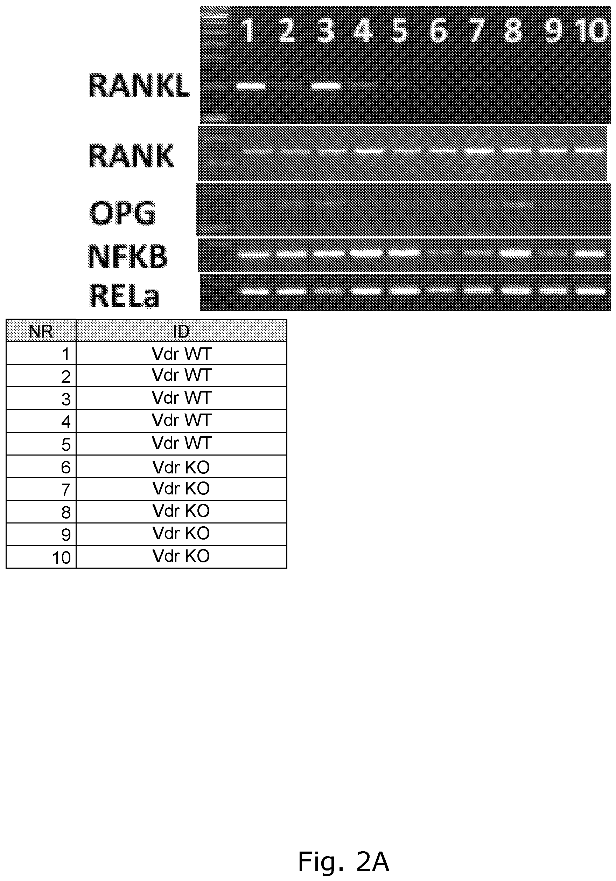

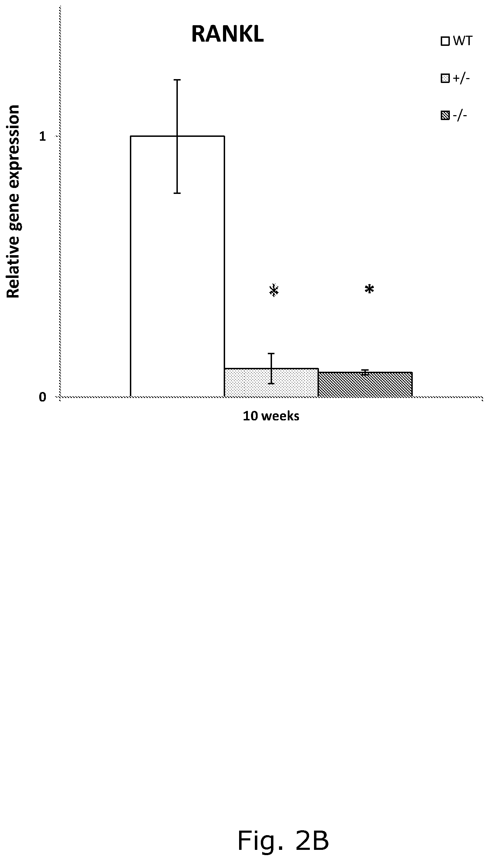

*Figure 2 (divided on two pages)*

Figure 2 A shows RT-PCR of selected transcripts from Swiss mice with a normal vitamin D receptor (Vdr wt) or with a mutated and inactive vitamin D receptor (Vdr KO). A primerset detects RANKL in normal mice, while the expression is low or absent in Vdr KO mice. RANK, OPG, RELa are expressed in all animals, while NFKB appears to be lower expressed in Vdr KO mice.

Figure 2 B shows quantitative PCR of RANKL from mice with a normal Vdr (wt), one inactive allelle (+/-) or two inactivated alleles (-/-). The significant downregulation of RANKL in both heterozygote and homozygote mutant mice indicates that the main isoform is RANKL1 as it is regulated by vitamin D, while isoform 3 is not.

*Figure 3*

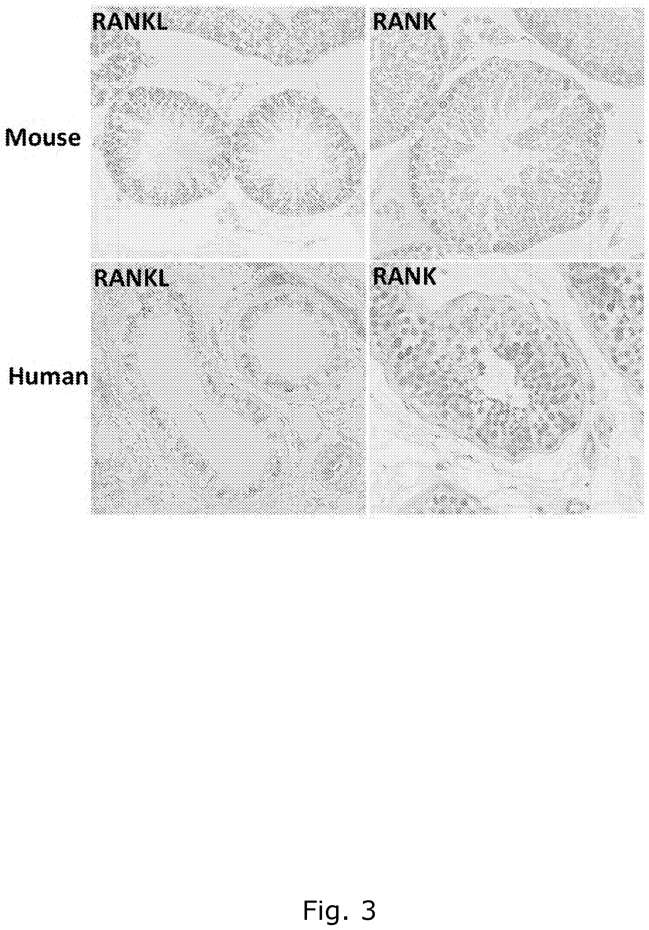

Figure 3 shows immunohistochemical expression of RANKL and RANK in normal testis and carcinoma *in situ* tubules obtained from humans besides expression in normal mouse testis. RANK is expressed in germ cells, while RANKL is expressed in Sertoli and later stages of germ cells.

*Figure 4*

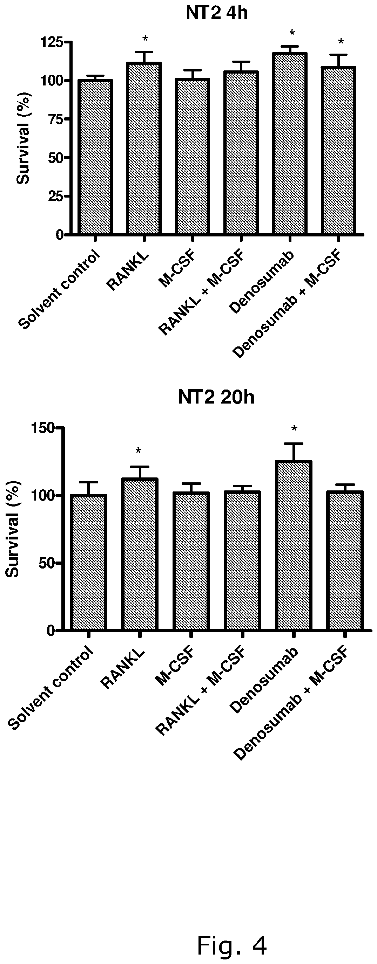

Figure 4: Survival/proliferation of NT2 cells after treatment for 20 hours, growth in normal media for 14 hours + 4 hours with resazurin dye. All treatments are at concentrations of 100 ng/ml. Values are mean $\pm$ std. (n=8), with * indicating significant difference $p < 0.05$ after t-test.

*Figure 5*
Figure 5: Survival/proliferation of TCam-2 cells after treatment for 4 and 20 hours, growth in normal media for 10 hours + 6,5 hours with resazurin dye. All treatments are at concentrations of 100 ng/ml. Values are mean $\pm$ std. (n=8), with * indicating significant difference $p < 0.05$ after t-test.

*Figure 6*

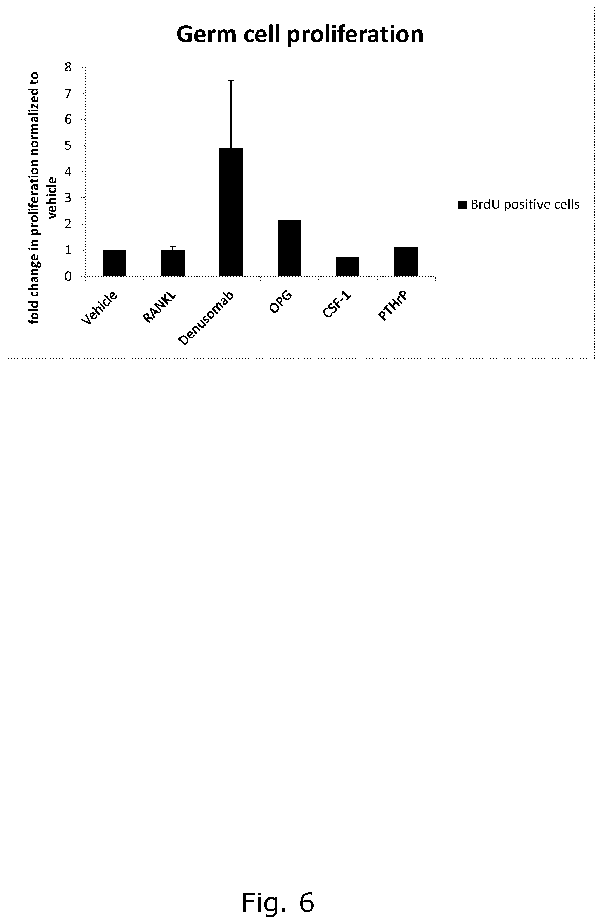

Figure 6 shows germ cell proliferaion in human ex vivo model. Germ cell proliferation was assessed by BrdU incorporation following 48 hours treatment of human testis specimens (N=3) with vehicle (DMSO), 100 ng/ml RANKL or 100 ng/ml Denosumab and also compared with one testis treated with 50 ng/ml OPG, 100 ng/ml CSF-1 and 100 ng/ml PTHrP. Data presented as mean change in proliferation + sem and data normalised to vehicle treated samles.

*Figure 7*

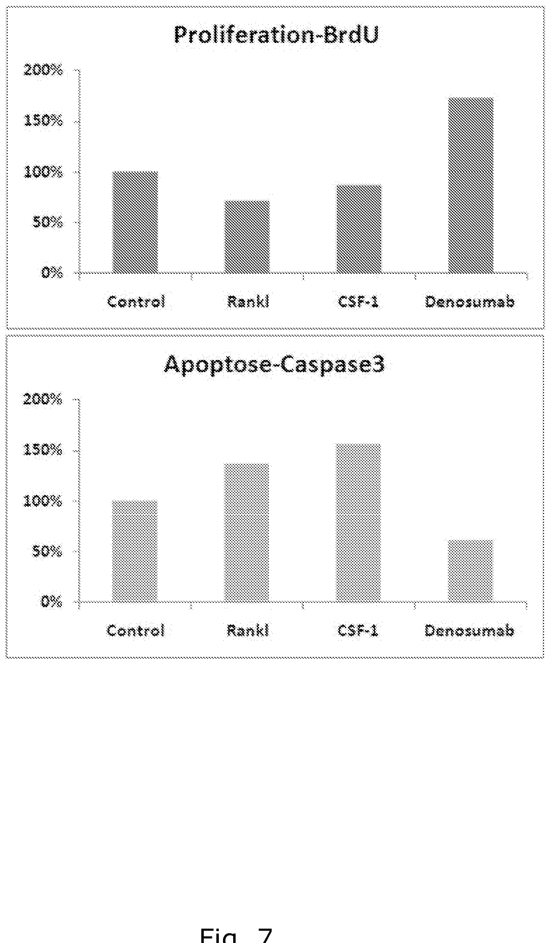

Figure 7 shows the effect on proliferation (top) and apoptosis (buttom) of treatment with RANKL, CSF-1, Denosumab and control in an ex vivo human testicular model containing Carcinoma *in situ* cells intratubularly. The mean number of germ cells expressing BrdU or Caspase 3 has been counted to compare the effect between the treatments.

*Figure 8*

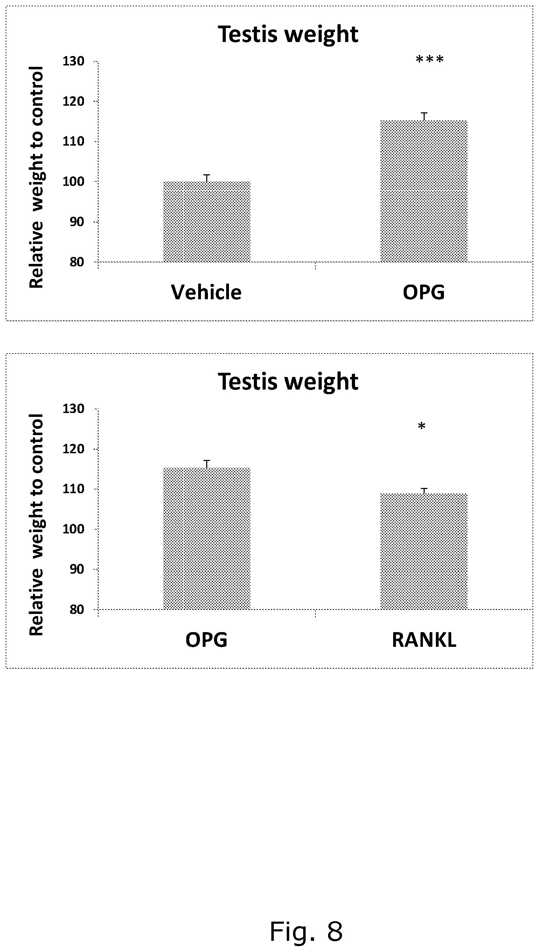

Figure 8 shows total testes weight in mice treated with vehicle, OPG or RANKL. Testis weight was assessed after 14 days treatment of 15 mice (5 mice randomized to each treatment :vehicle RANKL or OPG). Data presented as mean + sem and data normalised to vehicle treated samles. * mark $p < 0.05$ and *** $p < 0.001$.

*Figure 9*
Figure 9 shows total epididymal weight in mice treated with vehicle, OPG or RANKL. Testis weight was assessed after 14 days treatment of 15 mice (5 mice randomized to each treatment :vehicle RANKL or OPG). Data presented as mean + sem and data normalised to vehicle treated samles. NS mark $p > 0.05$ and *$p < 0.05$.

*Figure 10*

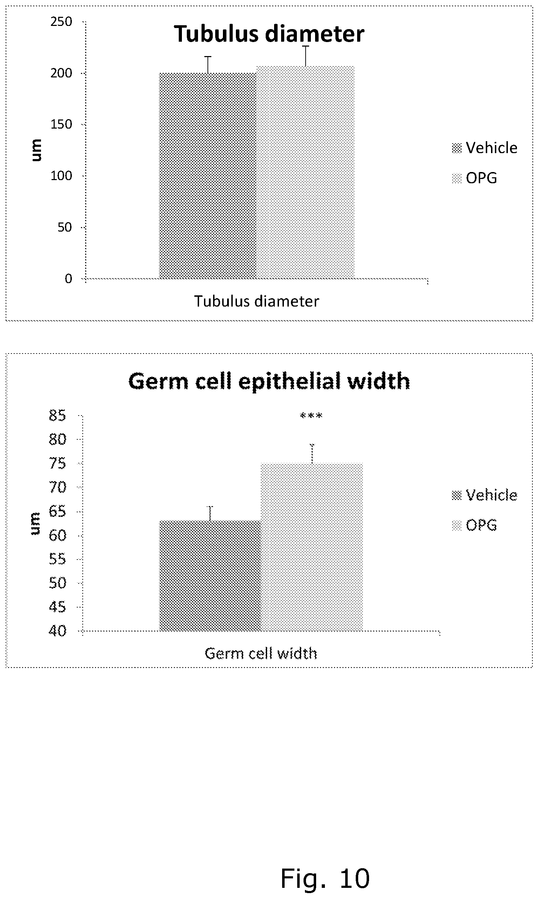

Figure 10 shows testicular morphometry in 10 week old mice. Testis from 10 old mice were fixed in Bouin and Seminiferous tubules with germ cell epithelia (stage 6- 12) were assessed (N=3 from each treatment arm).) Comparison of tubular diameter (top) and germ cell epithial width (buttom) in mice treated with vehicle or OPG for 14 days. Bars indicate mean $\pm$ s.e.m.

*Figure 11*
Figure 11 shows sperm count in mice treated with vehicle or OPG. Sperm count was assessed following miching of cauda epididymis in 1ml PBS was (5 mice randomized to each treatment vehicle or OPG). Data presented as mean + sem. * mark $p < 0.05$.

*Figure 12 (divided on two pages)*
Figure 12 shows RT-PCR of selected transcripts from human specimens marked from 1-23. Primersets detected the investigated isoforms of some bone signals in normal and pathological testicular specimens in addition to selected normal tissues and cell lines.

*Figure 13*

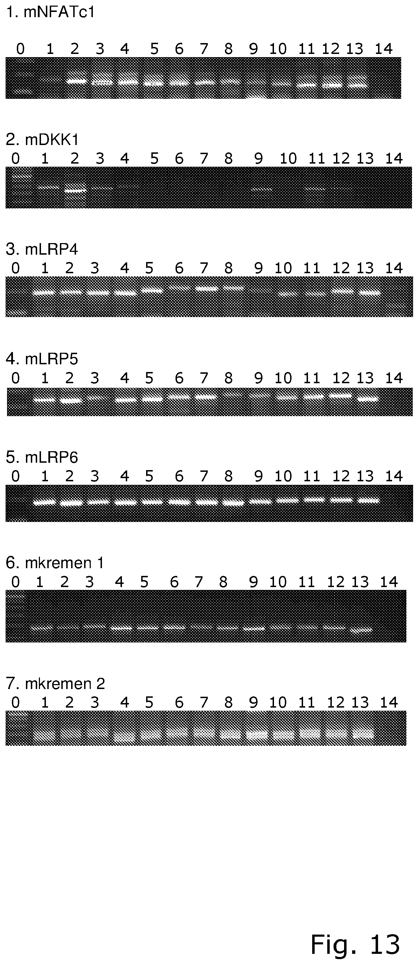

Figure 13 shows RT-PCR of selected transcripts from Swiss mice with a normal vitamin D receptor (Vdr wt) or with a mutated and inactive vitamin D receptor (Vdr -/-) or heterozygotes(Vdr -/+). 0=marker; 1-4= Vdr wt; 5-9= Vdr -/-; 10-13= Vdr -/+; and 14=blank. Specificprimersets detected NFATc1, DKK1, LRP4, LRP5, LRP6, kremen 1 and kremen 2in normal mice and for most of them also high expression level in Vdr KO mice. Interestingly, DKK1 which is a strong regulator of Wnt signaling and RANKL followed the expression pattern of RANKL, because the expression was much lower in Vdr KO mice compared with wildtype mice.

*Figure 14*

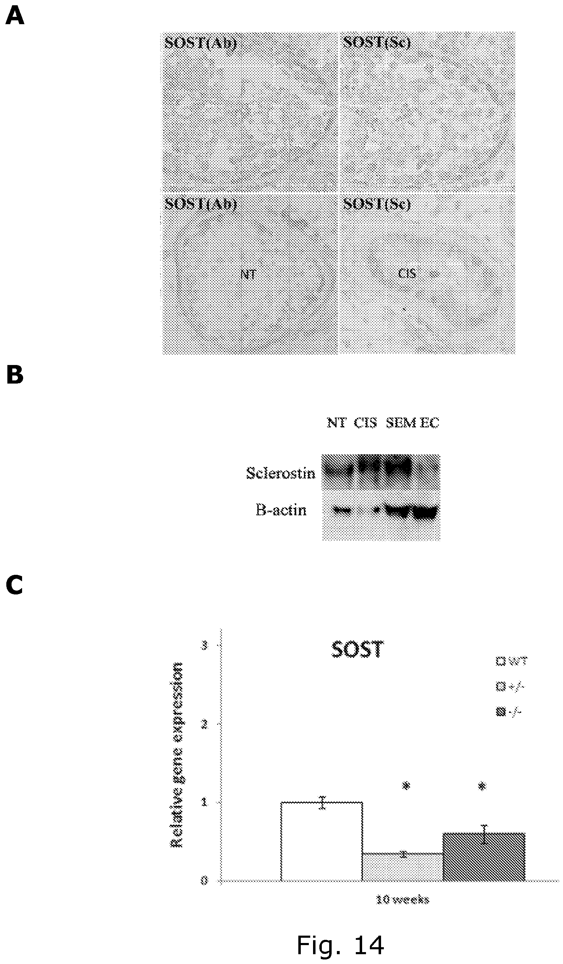

Figure 14 shows presence of Sclerostin in the male gonad. A) immunohistochemical expression of Sclerostin in the testis with expression in germ cells and peritubular cells by using two different antibodies in normal testis and carcinoma *in situ* tubules obtained from humans besides expression in normal mouse testis. B) WB confirmed the presence of sclerostin in normal testis, CIS, seminoma and Embryonal carcinoma. C) shows quantitative PCR of sclerostin (*SOST*) from mice with a normal Vdr (wt), one inactive allele (+/-) or two inactivated alleles (-/-). The significant downregulation of SOST in both heterozygote and homozygote mutant mice indicates that the main isoform of SOST is regulated by vitamin D.

*Figure 15*

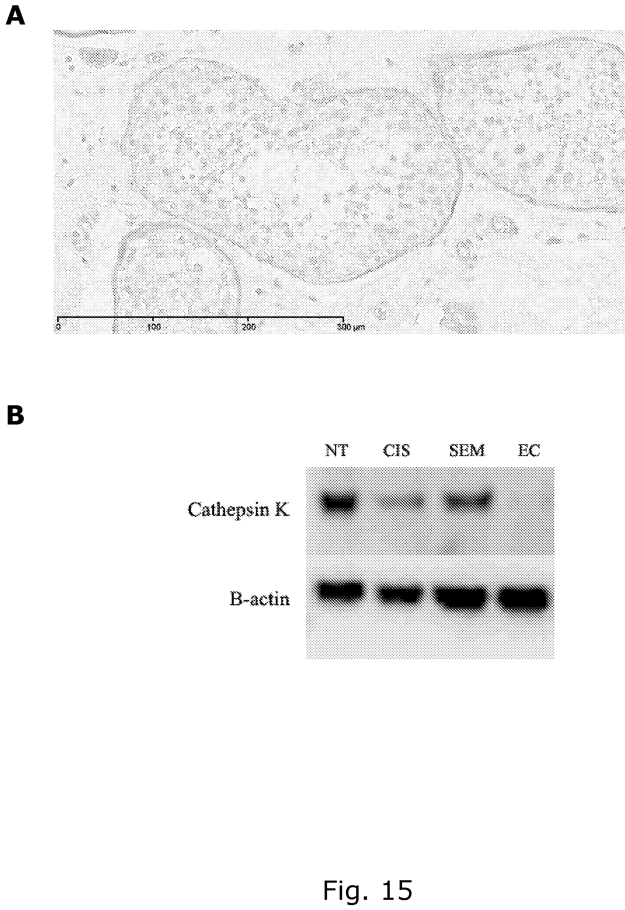

Figure 15 shows immunohistochemical expression of Cathepsin K in normal testis obtained from humans. A) Cathepsin is mainly expressed in peritubular cells but may also be expressed in some germ cells. B) WB confirms the presence of cathepsin K in normal testis, CIS and seminoma.

*Figure 16*
WB confirms the presence of PTH related peptid (PTHrP) and the PTH receptor in normal testis and CIS indicating that this regulator of RANKL signalling also may play a role for male reproductive health.

*Figure 17*
Overview
Germ cell proliferation and spermatogenesis depends on a complicated interpaly between interstitiel leydig cells and the cell residing in the tubular compartment comprising peritubular cells, Sertoli cells and the germ cells that eventually will develop into sperm. The crosstalk is complex and highly dependent on external stimuli from the environment for instance several hormones for instance LH and FSH. We have now evindece that classical bone signals are present in the seminiferous tubules and we believe there is striking similarities between paracrine signalling between stromal cells, osteoblasts and osteoclasts in the skeleton and peritubular, Sertoli and germ cells in the testis. OPG is able to block RANKL signalling from Sertoli cells or autocrine signalling from the germ cells and thereby inhibit RANK activition and subsequently downstreamt signalling mediated through NFKB, JNK, B-catenin, NFAT, TRAF etc. This influence germ cell proliferation in vitro and in vivo. Since OPG also partly block TRAIL may part of this phenomenon be mediated through TRAIL signalling. Several other bone specific ligands and receptors have been found by our group in the tubular compartment. This indicates that these regulators may influence the RANKL reponse and the cells involved in spermatogenesis. This may have clinical ptotential as some of the drugs available are able to block for instance Cathepsin K or Sclerostin.

*Figure 18*
Serum OPG level in fertile men, Left: OPG levels less than 4 pg/ml. Right: OPG levels above or equal to 4 pg/ml. Y-axis: Median sperm concentration.

*Figure 19*

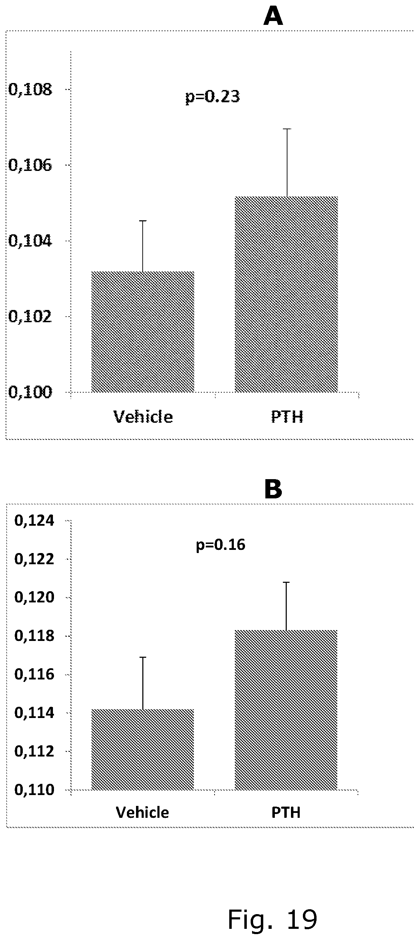

Figure 19 shows changes in testicular weight (gram) following injection of PTH for 2 weeks (A) or 5 weeks (B) compared with Vehicle. Data presented as mean + sem.

*Figure 20*

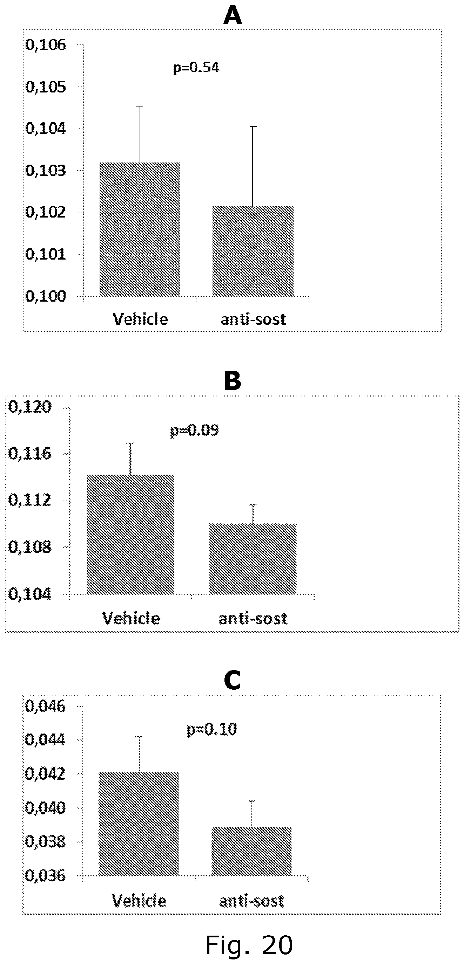

Figure 20 shows changes in testicular weight (gram) following injection of anti-sclerostin (anti-sost) for 2 weeks (A) or 5 weeks (B) compared with Vehicle. C: epididymal weight after 5 weeks treatment with vehicle or anti-sost.Data presented as mean + sem.

*Figure 21*

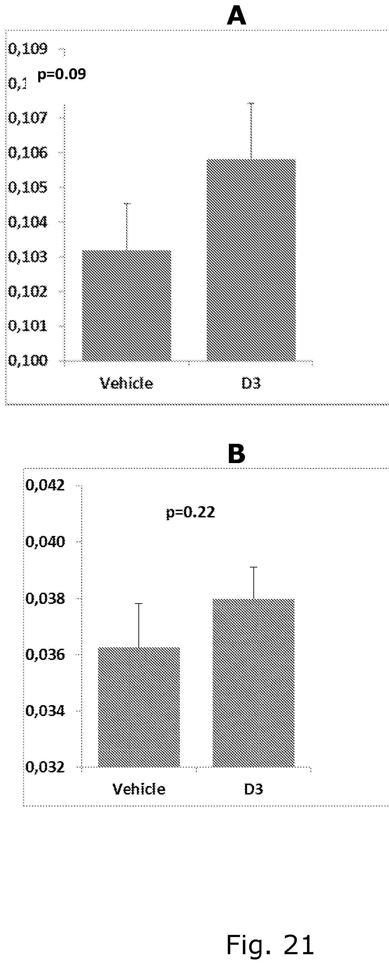

Figure 21 shows changes in A: testicular weight or B: Epididymal weight (in grams) following treatment with cholecalciferol (D3) for 2 weeks compared with Vehicle.Data presented as mean + sem.

*Figure 22*

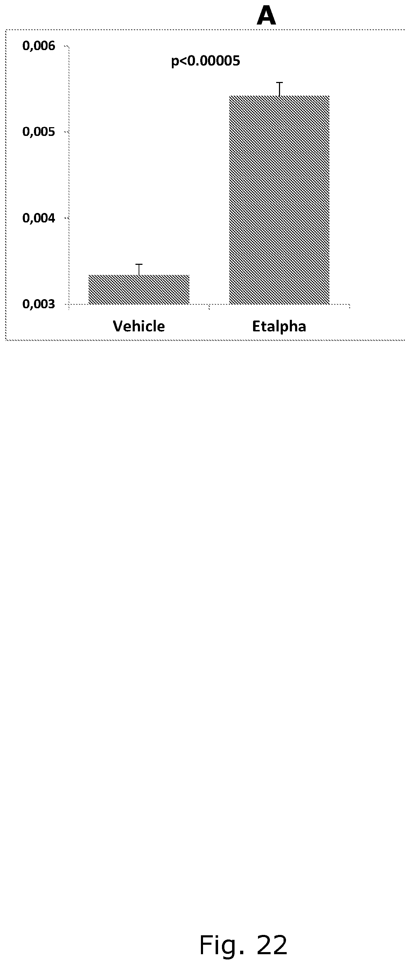

Figure 22 shows changes in A: testicular weight per total body weight (adjusted for body weight because the mice decreased significantly in body weight) following treatment with etalpha for 9 days compared with Vehicle. Data presented as mean + sem.

Figure 23

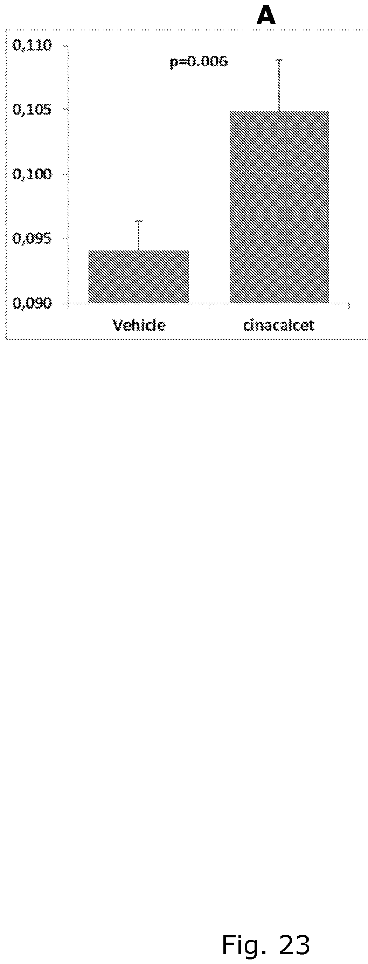

Figure 23 shows changes in A: testicular weight following treatment with cinacalcet for 2 weeks compared with Vehicle. Data presented as mean + sem.

Figure 24

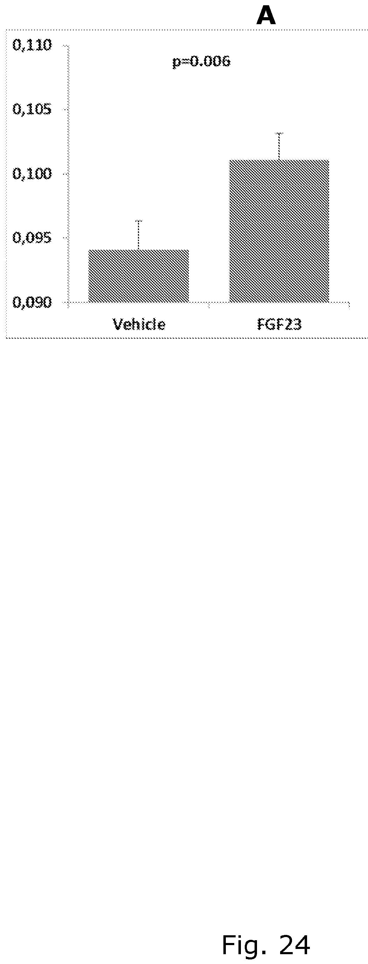

Figure 24 shows changes in A: testicular weight following treatment with FGF23 for 2 weeks compared with Vehicle. Data presented as mean + sem.

Figure 25

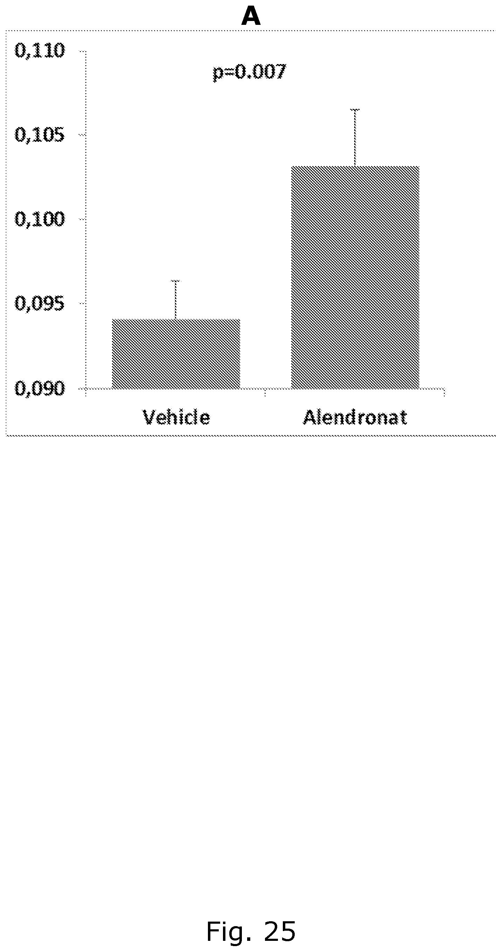

Figure 25 shows changes in A: testicular weight following treatment with alendronate for 2 weeks compared with Vehicle. Data presented as mean + sem.

Figure 26

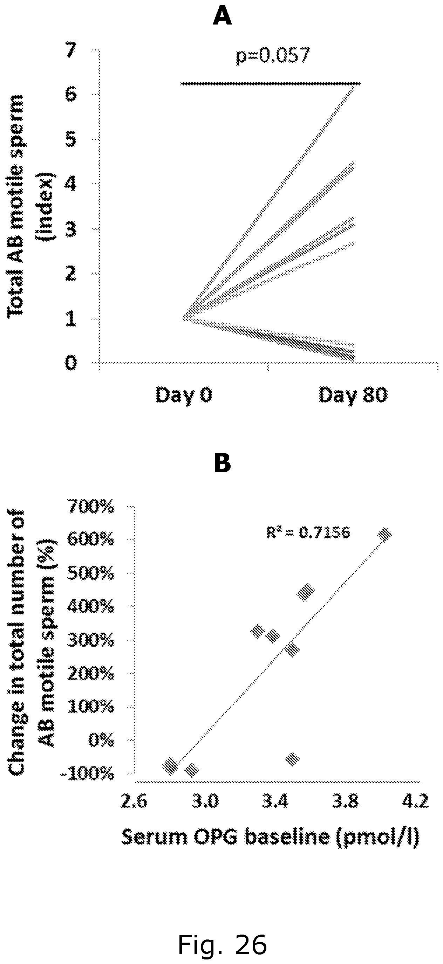

Figure 26 shows changes in total number of motile sperm (106) following treatment with Denosumab 60 mg. All men delivered 2 semen samples prior to treatment start. The average semen quality was determined at baseline. The semen sample delivered at day 80 was normalized to the same persons average value prior to treatment and presented as index (A). Afterwards the proportional change in semen quality as a function of serum OPG as baseline is shown (B). p=0.057

The present invention will now be described in more detail in the following.

## Detailed description of the invention

### Definitions

Prior to discussing the present invention in further details, the following terms and conventions will first be defined: Some of the antibodies and antigen binding domains of the invention bind selectively to RANKL/OPGbp that is they bind preferentially to RANKL/OPGbp with a greater binding affinity than to other antigens. The antibodies may bind selectively to human RANKL/OPGbp, but also bind detectably to non-human RANKL, such as murine RANKL. Alternatively, the antibodies may bind exclusively to human RANKL or RANK, with no detectable binding to non-human RANKL or RANK, respectively.

The term "monoclonal antibody" refers to an antibody obtained from a population of substantially homogeneous antibodies, wherein each monoclonal antibody will typically recognize a single epitope on the antigen. The term "monoclonal" is not limited to any particular method for making the antibody. For example, monoclonal antibodies of the invention may be made by the hybridoma method as described in Kohler et al. Nature 256, 495 (1975) or may be isolated from phage libraries using the techniques as described herein, for example.

The term "antigen binding domain" or "antigen binding region" or "fragment or derivative thereof" refers to that portion of the selective binding agent (such as an antibody molecule) which contains the amino acid residues that interact with an antigen and confer on the binding agent its specificity and affinity for the antigen. Preferably, the antigen binding region will be of human origin. In other embodiments, the antigen-binding region can be derived from other animal species, in particular domestic animal and rodents such as rabbit, rat or hamster.

The terms "effective amount" and "therapeutically effective amount" when used in relation to an antibody or antigen binding domain, fragment or derivative thereof, immunoreactive with a RANKL peptide, refer to an amount of a selective binding agent that is useful or necessary to support an observable change in the level of one or more biological activities of RANKL, wherein said change may be either an increase or decrease in the level of RANKL activity.

In the context of the present invention, the term "sequence identity" indicates a quantitative measure of the degree of homology between two amino acid sequences or between two nucleic acid sequences. If the two sequences to be compared are not of equal length, they must be aligned to give the best possible fit, allowing the insertion of gaps or, alternatively, truncation at the ends of the polypeptide sequences or nucleotide sequences. The sequence identity can be calculated as $\dfrac{\left(N_{ref} - N_{dif}\right) \cdot 100}{N_{ref}}$, wherein Ndif is the total number of non-identical residues in the two sequences when aligned and wherein Nref is the number of residues in one of the sequences. Hence, the DNA sequence AGTCAGTC will have a sequence identity of 75% with the sequence AATCAATC (Ndif=2 and Nref=8). A gap is counted as non-identity of the specific residue(s), i.e. the DNA sequence AGTGTC will have a sequence identity of 75% with the DNA sequence AGTCAGTC (Ndif=2 and Nref=8).

With respect to all embodiments of the invention relating to amino acid sequences or nucleotide sequences, the percentage of sequence identity between one or more sequences may also be based on alignments using the clustalW software (http:/www.ebi.ac.uk/clustalW/index.html) with default settings. For nucleotide sequence alignments these settings are: Alignment=3Dfull, Gap Open 10.00, Gap Ext. 0.20, Gap separation Dist. 4, DNA weight matrix: identity (IUB). For amino acid sequence alignments the settings are as follows: Alignment=3Dfull, Gap Open 10.00, Gap Ext. 0.20, Gap separation Dist. 4, Protein weight matrix: Gonnet.

Alternatively, nucleotide sequences may be analysed using programme DNASIS Max and the comparison of the sequences may be done at http://www.paralign.org/. This service is based on the two comparison algorithms called Smith-Waterman (SW) and ParAlign. The first algorithm was published by Smith and Waterman (1981) and is a well-established method that finds the optimal local alignment of two sequences. The other algorithm, ParAlign, is a heuristic method for sequence alignment; details on the method are published in Rognes (2001). Default settings for score matrix and Gap penalties as well as E-values were used.

*Romosozumab*

Romosozumab (AMG 785) is a humanized monoclonal antibody that targets sclerostin e.g. for the treatment of osteoporosis. CAS number:909395-70-6.

*Odanacatib*

Odanacatib (pINN; codenamed MK-0822) is an investigational treatment drug for osteoporosis and bone metastasis. It is an inhibitor of cathepsin K, an enzyme known to be involved in bone resorption. Systematic (IUPAC) name: N-(1-cyanocyclopropyl)-4-fluoro-N2-{(1S)-2,2,2-trifluoro-1-[4'-(methylsulfonyl)biphenyl-4-yl]ethyl}-L-leucinamide. CAS number: 603139-19-1.

*VEL-0230*

The drug, VEL-0230, alternatively named NC-2300, is a highly specific, irreversible inhibitor of bone Cathepsin K developed by Velcura Therapeutics. For further details see Asagiri M et al. (2008). Cathepsin K-dependent toll-like receptor 9 signaling revealed in experimental arthritis. Science, 319(5863), 624-627 and Hussein, H. et al. (2014). Pharmacokinetics and bone resorption evaluation of a novel Cathepsin K inhibitor (VEL-0230) in healthy adult horses. Journal of veterinary pharmacology and therapeutics. Vel-0230 or sodium (2S,3S)-3-(((S)-1-isobutoxy-4-methylpentan-2-yl)carbamoyl)oxirane-2-carboxylate exists in a soidum bound and free form $C14H24NNaO5$ or $C14H25NO5$, respectively. There exist other Cathepsin K inhibitors, but not all of them are commercial available. For instance 1-{1-[4-fluoro-2-(2-oxo-pyrrolidin-1-yl)-phenyl]-3-oxo-piperidin-4-ylcarbamoyl}-cyclohexyl)-amide (OST-4077).

*$C_{75}H_{118}N_{20}O_{22}S$ and cyclosporine A*

$C_{75}H_{118}N_{20}O_{22}S$ and cyclosporine A are inhibitors of NFAT(1). cyclosporine A has CAS No. 59865-13-3

$C_{75}H_{118}N_{20}O_{22}S$ has CAS No. 249537-73-3.

*DKN-01 and/or BHQ880*

DKN-01 and BHQ880 are both inhibitors of DKK1.

DKN-01 is a humanized monoclonal antibody directed against Wnt antagonist Dickkopf-1 (DKK1) with potential anti-osteolytic activity. DKK1-neutralizing monoclonal antibody DKN-01 binds to and inhibits DKK1, which restores Wnt pathway signaling. NCI Thesaurus Code: C113331, NCI Metathesaurus CUI: CL458159. Source: National Cancer Institute (NCI) (NCI Thesaurus).

BHQ880 is a humanized monoclonal antibody directed against Wnt antagonist Dickkopf-1 (DKK1) with potential anti-osteolytic activity. Anti-DKK1 monoclonal antibody BHQ880 binds to and inhibits DKK1, enhancing signaling through the Wnt pathway, which may result in osteoblast differentiation and activation within the bone matrix and the reversal of tumor-induced osteolytic disease. DKK1 is a potent Wnt signaling pathway antagonist. NCI Thesaurus Code: C80040. PDQ Closed Trial Search ID: 614254. Source: National Cancer Institute (NCI) (NCI Thesaurus).

*Fertility/fertility potential*

In general, if a person has a high sperm number (above 40 millions), above 50% motile sperm and more than 12 % morphological normal spermatozoa the person is regarded to have a normal fertility potential. This is not an accurate estimate of male fertility potential and the result of the semen analysis does not always correlate with the ability to have children. This is also illustrated by the fact that WHO recently has changed the reference levels as a large proportion of the male population otherwise would be regarded as having a fertility potential below normal. No precise method for predicting the male fertility potential exists presently.

More than 9 % of all babies are born after assisted reproductive methods in Denmark, and the incidence of infertility is increasing worldwide. About 50% of cases are attributable to the male partner and the first step in the clinical evaluation involves semen analysis to evaluate male fertility potential. The clinicians use the analysis in deciding the appropriate reproductive method.

It is estimated that some 1.400.000 semen analyses are performed each year worldwide.

Semen analysis is currently a cumbersome and lengthy process. It is performed by standard microscopy and is based on the subjective analysis of three key parameters: motility, morphology, and total sperm number. The analysis has several drawbacks as it necessitates a trained medical staff, presence of the patient with a fresh semen sample (less than 1 hour old), laboratory facilities etc. making it a costly and time consuming procedure. Logistically, the analysis requires the patient to deliver a semen sample at the laboratory where the testing is performed, which for many patients is considered a major embarrassment. Furthermore, semen analyses using the current methods is highly prone to intra- and inter observer variability due to the subjective assessment of the key parameters, which severely can affect the utility of the analysis, whereby the clinician is troubled in the guidance of the couple to the relevant assisted reproductive method.

Due to above mentioned problems with the currently used methods, a large need exist for a new method for semen analysis, which accurately and objectively can assess male fertility potential in order to stratify patients into the correct reproductive method. The problems with the existing analysis are illustrated by the latest WHO recommendations from 2010, where the reference values for normal semen analysis were changed and caused a huge debate in fertility and andrology meetings worldwide.

***Compound for medical use***

The nucleotide sequences and predicted amino acid sequences of murine and human RANKL are shown in SED ID NO: 1 and 3, respectively. The murine and human RANKL amino acid sequences are furthermore shown in SEQ ID NO 2 and 4, respectively.

Computer analysis of the RANKL sequence has indicated that RANKL is a Type 2 transmembrane protein. Thus, the murine RANKL protein contains, as predicted, an intracellular domain of 47 amino acids, a transmembrane domain of 21 amino acids, and an extracellular domain of 247 amino acids. The human RANKL contains, as predicted, an intracellular domain of 47 amino acids, a transmembrane domain of 21 amino acids, and an extracellular domain of 249 amino acids.

Soluble RANKL comprises a signal peptide and the extracellular domain or a fragment thereof.

RANKL is similar to other members of the TNF family having a region of amino acids between the transmembrane domain and the receptor binding domain, which does not seem to be required for biological activity. Based on alignment of the amino acid sequence, it is contemplated that the receptor binding domain of human RANKL is from about amino acid 162 to about amino acid 317 of SEQ ID NO: 4 starting with a highly conserved Ala residue amongst many family members, i.e. amino acid 162 of SEQ ID NO: 4. The murine receptor binding domain is from about amino acid 139 to about amino acid 294 of SEQ ID NO: 2.

However, the person skilled in the art would recognise that the actual receptor binding domain may be different from that predicted by alignment and computer analysis.

The N-terminal amino acid of a soluble RANKL polypeptide is expected to be within about five amino acids of the conserved Ala residue on either side. Alternatively, all or a part of the spacer region may be included in the N-terminus of the soluble RANKL peptide, as may the all or part of the transmembrane and/or intracellular domains, provided that the resulting soluble RANKL is not membrane-associated.

Thus, a soluble RANKL polypeptide will have an N-terminal amino acid selected from the group consisting of amino acids 1 through 162 of SEQ ID NO: 4 corresponding to amino acids 1 to 139 of SEQ ID NO: 2. Alternatively, the N-terminal amino acid is between 69 and 162 of SEQ ID NO: 4 (human RANKL) corresponding to amino acids 48 to 139 of SEQ ID NO: 2 (murine RANKL). Likewise, the C-terminal amino acid can be between amino acid 313 and 317 of SEQ ID NO: 4.

Since the discovery of the OPG-RANK-RANKL signalling pathway several inhibitors of RANKL function and signalling have been evaluated and/or developed as candidates for clinical use. A review of the development of RANKL inhibitors is provided in Lacey et al., Nature Reviews, (2012), Vol. 11, 401-419. These inhibitors include recombinant, full length OPG and RANK-Fc, which is a fusion protein comprising the four extracellular CRDs of RANK and the Fc-portion of human IgG.

*Sclerostin (SOST)*

The current data indicate that Sclerostin (SOST) antagonists (e.g. the antibody Romosozumab) can also be used to regulate germ cell proliferation and therefore be used to treat male infertility or as an contraceptive depending on the use of antagonist or agonist for instance sclerostin versus anti-sclerostin. For example the current data shows that Sclerostin and the corresponding receptor is expressed on both RNA and protein level in the testis from both mouse and man (figures 12 and 14). Without being bound by theory, this clearly indicates that Sclerostin (SOST) antagonists may be used to treat male infertility and on the other hand promoters of Sclerostin or the compound it self may be used as male contraception as, SOST directly antagonizes the activity of BMP2, BMP4, BMP6 and BMP7 in a dose-dependent manner and therefore may increase RANKL signalling and impair germ cell proliferation

In example 10 (and figure 20), it is shown that an antibody against sclerostin inhibits sperm formation in mice. Example 10 shows data with an antibody against sclerostin, indicating that the opposite effect may be obtained by providing sclerostin.

*Cathepsin k*

The current data indicate that Cathepsin K inhibitors (such as Odanacatib, OST-4077 and VEL-0230) can also be used to regulate germ cell proliferation and therefore be used to treat male infertility or as an contraceptive depending on the use of antagonist or agonist for instance Cathepsin K versus anti-Cathepsin K. The current data shows that Cathepsin K is expressed at both RNA and protein level in testis. It appears to be highly expressed in peritubular cells and germ cells, which makes it accessible to exogenous treatment. As in the skeleton it is likely that Cathepsin K in the germ cell may be under direct influence of RANKL and Cathepsin K in peritubular cells indirectly may be connected with the testicular RANKL system. Without being bound by theory, this clearly indicates that Cathepsin K inhibitors) antagonists such as Odanacatib, OST-4077 and VEL-0230 may be used to treat male infertility.

*NFAT*

The current data indicate that Nuclear factor of activated T cells (NFAT) inhibitors (such as $C_{75}H_{118}N_{20}O_{22}S$ or cyclosporine A) can also be used to regulate germ cell proliferation and therefore be used to treat male infertility or as an contraceptive depending on the use of antagonist or agonist for instance NFAT versus anti-NFAT. For example the current data shows that the most important proteins NFATc1, NFATc2, and maybe even more such as NFATc3, NFATc4, and NFAT5 are expressed in the testis. NFAT is involved in downstream signaling of RANKL and essential for osteoclasotgenesis, which is suggestive for a central effect on spermatogenesis. Without being bound by theory, this clearly indicates that NFAT inhibitors may be used to treat male infertility.

*Dickkopf-related protein 1 (DKK1), WNT, Kremen, Frizzled and B-catenin.*

The current data indicate that DKK1, DKK2, WNT, Kremen, Frizzled inhibitors among others can also be used to regulate germ cell proliferation and therefore be used to treat male infertility or as an contraceptive depending on the use of antagonist or agonist for instance DKK1 versus anti-DKK1. For example the current data shows that DKK1 is expressed in mouse and human testis. Interestingly DKK1 is heavily regulated by vitamin D and the expression level vanishes in Vitamin D receptor Knock out mice as observed for SOST and RANKL. Highly supportive for a similar function as they are heavily interconnected in bone and potentially also in the gonad. Without being bound by theory, this clearly indicates that inhibitors DKK1, DKK2, WNT, Kremen, and/or Frizzled may be used to treat male infertility. Known inhibitors of DKK1 are DKN-01 and BHQ880.

*Parathyroid hormone (PTH) and PTH related peptid (PTHrP)*

As shown in Figure 16, WB confirms the presence of PTH related peptid (PTHrP) and the PTH receptor in normal testis and CIS indicating that this regulator of RANKL signalling also may play a role for male reproductive health. It is known that PTH and PTHrP is a powerful regulator of bone cell interaction and RANKL signaling. Local PTHrP production may therefore be important for regulating germ cell proliferation. See also example 9 and figure 19.

*CHOLECALCIFEROL, ETALPHA, PTH, CINACALCET, FGF23, and BISPHOSPHONATES*

To further elucidate the use of compounds targeting the expected sperm formation pathway, different compounds were tested in mice models.

Data for PTH can be found in example 9.

Data for CHOLECALCIFEROL can be found in example 11.

Data for ETALPHA can be found in example 12.

Data for CINACALCET can be found in example 13.

Data for FGF23 can be found in example 14. FGF23 binds to Klotho.

Data for BISPHOSPHONATES (ALENDRONATE) can be found in example 15.

*Antibodies or fragments thereof capable of binding RANKL*

The compound, which is immunoreactive with an OPGbp/RANKL peptide, such as said antigen binding domain, said fragment of an antibody, said antigen binding domain or said derivative of an antibody or an antigen binding domain of the invention may be an antagonist compound, which decreases the level of at least one biological activity of RANKL. Antagonist antibodies of RANKL may also be referred to as inhibitory or neutralizing antibodies of RANKL.

An antibody substantially inhibits binding of a ligand to a receptor when an excess of antibody reduces the quantity of receptor bound to counter-receptor or ligand by at least about 20%, 40%, 60%, 80%, 85%, 90% or more (as measured in an *in vitro* competitive binding assay).

The terms "heavy chain" and "light chain" of said antibody each includes any polypeptide having sufficient variable region sequence to confer specificity for an OPGbp/RANKL peptide. A full-length heavy chain includes a variable region domain, VH, and three constant region domains, CH1, CH2, and CH3. The VH domain is at the amino-terminus of the polypeptide, and the CH3 domain is at the carboxy-terminus. The term "heavy chain", as used herein, encompasses a full-length heavy chain and fragments thereof. A full-length light chain includes a variable region domain, VL, and a constant region domain, CL. Like the heavy chain, the variable region domain of the light chain is at the amino-terminus of the polypeptide. The term "light chain", as used herein, encompasses a full length light chain and fragments thereof. A Fab fragment is comprised of one light chain and the CH1 and variable regions of one heavy chain. The heavy chain of a Fab molecule cannot form a disulfide bond with another heavy chain molecule. A Fab' fragment contains one light chain and one heavy chain that contains more of the constant region, between the CH1 and CH2 domains, such that an interchain disulfide bond can be formed between two heavy chains to form a F (ab') 2 molecule. The Fv region comprises the variable regions from both the heavy and light chains, but lacks the constant regions. Single-chain antibodies are Fv molecules in which the heavy and light chain variable regions have been connected by a flexible linker to form a single polypeptide chain which forms an antigen-binding region. Single chain antibodies are discussed in detail in WO 88/01649 and U.S. Patent Nos. 4,946,778 and 5,260,203.

As described above, anti-RANKL/OPGbp antibodies and antigen binding domains which inhibit at least one activity of OPBbp/RANKL have been identified.

Polyclonal antibodies are heterogeneous populations of antibody molecules derived from the sera of animals immunized with an antigen. An antigen is a molecule or a portion of a molecule capable of being bound by an antibody which is additionally capable of inducing an animal to produce antibody capable of binding to an epitope of that antigen. An antigen can have one or more epitopes. The specific reaction referred to above is meant to indicate that the antigen will react, in a highly selective manner, with its corresponding antibody and not with the multitude of other antibodies which can be evoked by other antigens.

Polyclonal antibodies directed toward a RANKL polypeptide generally are raised in animals (e.g., rabbits or mice) by multiple subcutaneous or intraperitoneal injections of RANKL and an adjuvant. In accordance with the invention, it may be useful to conjugate an RANKL polypeptide, or a variant, fragment, or derivative thereof to a carrier protein that is immunogenic in the species to be immunized, such as keyhole limpet heocyanin, serum, albumin, bovine thyroglobulin, or soybean trypsin inhibitor. Also, aggregating agents such as alum are used to enhance the immune response. After immunization, the animals are bled and the serum is assayed for anti-RANKL antibody titer.

Monoclonal antibodies (mAbs) contain a substantially homogeneous population of antibodies specific to antigens, which population contains substantially similar epitope binding sites. Such antibodies may be of any immunoglobulin class including IgG, IgM, IgE, IgA, IgD and any subclass thereof. A hybridoma producing a monoclonal antibody of the present invention may be cultivated *in vitro, in situ,* or *in vivo.* Production of high titers *in vivo* or *in situ* is a preferred method of production.

Monoclonal antibodies directed toward OPGbp/RANKL are produced using any method which provides for the production of antibody molecules by continuous cell lines in culture. Examples of suitable methods for preparing monoclonal antibodies include hybridoma methods of Kohler et al., Nature 256, 495-497 (1975), and the human B-cell hybridoma method, Kozbor, J. Immunol. 133, 3001 (1984); Brodeur et al., Monoclonal Antibody Production Techniques and Applications, pp. 51-63 (Marcel Dekker, Inc., New York, 1987); and Harlow and Lane, Antibodies: A Laboratory Manual, Cold Spring Harbor Laboratory (1988).

A particularly preferred method for producing monoclonal antibodies directed towards OPGbp/RANKL involves immunizing the XenoMouse as described in Green, LL, J. Immunol. Methods (1999), Vol. 231, 11-25, with a OPGbp/RANKL peptide, such as a full-length human RANKL protein.

Preferred anti-RANKL or anti-RANK antibodies include monoclonal antibodies which will inhibit partially or completely the binding of human RANKL to its cognate receptor, RANK, or an antibody having substantially the same specific binding characteristics, as well as fragments and regions thereof. Preferred methods for determining monoclonal antibody specificity and affinity by competitive inhibition can be found in Harlow et al., Antibodies: A Laboratory Manual, Cold Spring Harbor Laboratory Press, Cold Spring Harbor, N.Y., 1988), Colligan et al., eds., Current Protocols in Immunology, Greene Publishing Assoc and Wiley Interscience, N.Y., (1992, 1993), and Muller, Meth. Enzymol., 92:589-601 (1983).

Chimeric antibodies are molecules in which different portions are derived from different animal species, such as those having a variable region derived from a murine monoclonal antibody and a human immunoglobulin constant region. Chimeric antibodies are primarily used to reduce immunogenicity in application and to increase yields in production, for example, where murine monoclonal antibodies have higher yields from hybridomas but higher immunogenicity in humans, such that human/murine chimeric monoclonal antibodies are used. Chimeric antibodies and methods for their production are known in the art. See Cabilly et al., Proc. Natl. Acad. Sci. USA, 81:3273-3277 (1984); Morrison et al., Proc. Natl. Acad. Sci. USA, 81:6851-6855 (1984); Boulianne et al., Nature, 312:643-646 (1984); Neuberger et al., Nature, 314:268-270 (1985); Liu et al., Proc. Natl. Acad. Sci. USA, 84:3439-3443 (1987); and Harlow and Lane Antibodies: A Laboratory Manual, Cold Spring Harbor Laboratory (1988).

For example, chimeric monoclonal antibodies of the invention may be used as a therapeutic. In such a chimeric antibody, a portion of the heavy and/or light chain is identical with or homologous to corresponding sequences in antibodies derived from a particular species or belonging to one particular antibody class or subclass, while the remainder of the chain(s) is identical with or homologous to corresponding sequence in antibodies derived from another species or belonging to another antibody class or subclass, as well as fragments of such antibodies, so long as they exhibit the desired biological activity (see U.S. Patent No. 4, 816, 567 ; Morrison et al., Proc. Natl. Acad. Sci. , 81, 6851-6855 (1985).

The term "chimeric antibody", as used herein, includes monovalent, divalent or polyvalent immunoglobulins. A monovalent chimeric antibody is a dimer (HL) formed by a chimeric H chain associated through disulfide bridges with a chimeric L chain. A divalent chimeric antibody is tetramer (H2L2) formed by two HL dimers associated through at least one disulfide bridge. A polyvalent chimeric antibody can also be produced, for example, by employing a CH region that aggregates (e.g., from an IgM H chain, or [micro] chain).

Murine and chimeric antibodies, fragments and regions of the present invention may comprise individual heavy (H) and/or light (L) immunoglobulin chains. A chimeric H chain comprises an antigen binding region derived from the H chain of a non-human antibody specific for RANKL, which is linked to at least a portion of a human H chain C region (CR), such as CH1 or CH2.

A chimeric L chain according to the present invention comprises an antigen binding region derived from the L chain of a non-human antibody specific for RANKL, linked to at least a portion of a human L chain C region (CL).

Selective binding agents, such as antibodies, fragments, or derivatives, having chimeric H chains and L chains of the same or different variable region binding specificity, can also be prepared by appropriate association of the individual polypeptide chains, according to known method steps, e.g., according to Ausubel et al., eds. Current Protocols in Molecular Biology, Wiley Interscience, N.Y. (1993), and Harlow et al., Antibodies: A Laboratory Manual, Cold Spring Harbor Laboratory Press, Cold Spring Harbor, N.Y. (1988). With this approach, hosts expressing chimeric H chains (or their derivatives) are separately cultured from hosts expressing chimeric L chains (or their derivatives), and the immunoglobulin chains are separately recovered and then associated. Alternatively, the hosts can be co-cultured and the chains allowed to associate spontaneously in the culture medium, followed by recovery of the assembled immunoglobulin, fragment or derivative.

*Determining the fertility potential in a male subject*

In an aspect the invention relates to a method for determining a likely effect of a treatment to improve male fertility, the method comprising

• determining the level of OPG in a blood serum sample, which has been obtained from a male subject;

• comparing said determined OPG level to a reference level; and

• determining that said subject will

∘ likely not benefit from treatment with an antagonist or inhibitor of RANKL, if said level is lower than said reference level, or

∘ likely benefit from treatment with an antagonist or inhibitor of RANKL, if said level Is equal to higher than said reference level;

wherein said reference level is above 3 pmol/l OPG in blood serum.

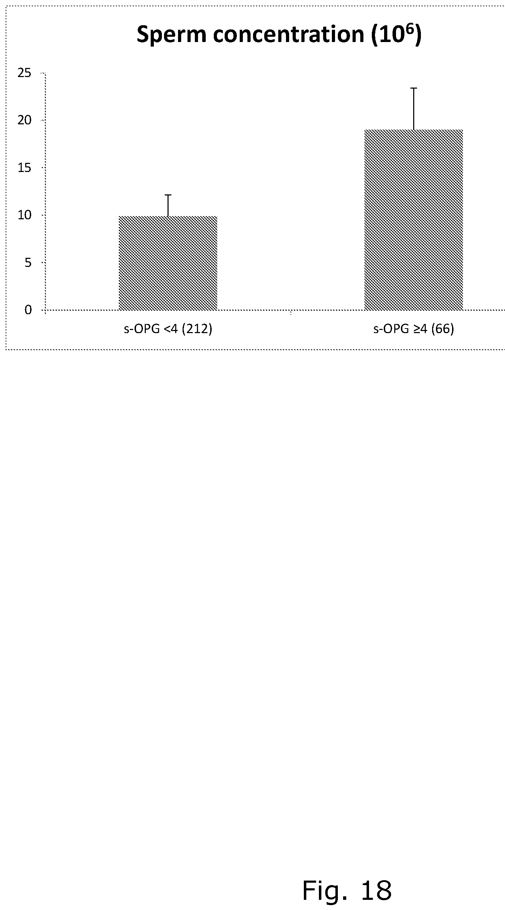

Example 7 clearly shows that the levels of serum OPG is a determining factor for sperm production in infertile men. 300 infertile men delivered two semen samples and had a blood sample drawn. The infertile men with serum OPG level above 4 had almost two fold higher median sperm concentration compared with men with serum OPG<4 pg/ml as shown in Figure 18. If we stratified the men in 20% percentiles instead the data were corroborated indicating that high OPG and as a result increased testicular RANKL inhibition is associated with increased sperm production and thus in line with in vitro, ex vivo and in vivo data presented in Figures 5-11.

Surprisingly, it was also found that for the treatment to be efficient (exemplified by the use if Denosumab), a positive change in the number of motile sperm, was more pronounced in subjects having a relatively high baseline blood serum levels of OPG. In example 8 and figure 26 it is shown that OPG is a good marker to use for determining the potential of treatment with a compound or composition according to the invention. A threshold level could e.g. be somewhere in the range 3 to 4, such as 3 to 3.5 pmol/l. Thus, a threshold level may also be a range, where both levels higher and lower a certain range would not be beneficial.

In sum

- OPG serum levels above 4 pmol/l may be indicative of normal fertility,
- serum levels below 3 pmol/l may be indicative of that the treatment will not to working (in worst case have the opposite effect); and
- an OPG serum level above 3 pmol/l (such as in the range 3 to 5 or 3 to 4 pmol/l) may be the preferred group to treat according to the invention.

Thus, it is to be understood that the threshold level for determining/indicating normal fertility (first threshold level) is not necessarily the same as the threshold level (second threshold level) for determining/indicating that a treatment will be efficient.

A couple is normally considered to be infertile if the couple has not conceived after 12 months of contraceptive-free intercourse. The fertility potential in a male mammal should be understood as the ability of said male's sperm to accomplish fertilization or to fertilize the egg. The male fertility potential is normally evaluated by semen analysis. Thus the fertility potential in a male mammal depends on semen quality as semen quality correlates with the ability of semen to accomplish fertilization. Semen quality depends on both sperm quantity and quality. Decreased semen quality is a major factor of male infertility.

A semen analysis evaluates certain characteristics of a male's semen and the sperm contained in the semen. It may be done while investigating a couple's infertility. It is also used in stud farming, dog feeding and farm animal breeding. The characteristics measured by the current tests for semen analysis are only some of the factors in semen quality. The most common characteristics measured to evaluate sperm quality are: sperm count, motility, morphology, volume, fructose level and pH.

Thus the current method to predict the fertility potential in a male mammal is by measuring sperm count, motility and morphology. The World Health Organization (WHO) has until recently defined that a male individual has a normal fertility potential if the semen analysis shows a sperm number above 40 millions, more than 50% motile sperm and more than 12% morphological normal spermatozoa. WHO has, however, now changed this definition and lowered the reference values as a high proportion of the male population otherwise would be regarded as having a fertility potential below normal.

The semen analysis is however not an accurate estimate of male fertility potential and has been shown not always to correlate with the ability of the semen to accomplish fertilization or the ability of the semen to fertilize the egg.

The chosen reference level may be changed if desiring a different specificity or sensitivity as known in the art. Similar, the skilled person would know of different reliable analytical tools when it comes to handling multible markers simultaneously. Such tools include artificial neural networks, logistic regression, classification tree and mixture discrimant analysis.

*Sensitivity*

As used herein the sensitivity refers to the measures of the proportion of actual positives which are correctly identified as such - in analogy with a diagnostic test, i.e. the percentage of mammals or people having a fertility potential below normal who are identified as having a fertility potential below normal.

Usually the sensitivity of a test can be described as the proportion of true positives of the total number with the target disorder i.e. a fertility potential below normal. All patients with the target disorder are the sum of (detected) true positives (TP) and (undetected) false negatives (FN).

*Specificity*

As used herein the specificity refers to measures of the proportion of negatives which are correctly identified - i.e. the percentage of mammal with a normal fertility potential that are identified as not having a fertility potential below normal. The ideal diagnostic test is a test that has 100 % specificity, i.e. only detects mammal with a fertility potential below normal and therefore no false positive results, and 100 % sensitivity, and i.e. detects all mammals with a fertility potential below normal and therefore no false negative results.

For any test, there is usually a trade-off between each measure. For example in a manufacturing setting in which one is testing for faults, one may be willing to risk discarding functioning components (low specificity), in order to increase the chance of identifying nearly all faulty components (high sensitivity). This trade-off can be represented graphically using a ROC curve.

Selecting a sensitivity and specificity it is possible to obtain the optimal outcome in a detection method. In determining the discriminating value distinguishing mammals having a fertility potential below normal, the person skilled in the art has to predetermine the level of specificity. The ideal diagnostic test is a test that has 100% specificity, i.e. only detects mammals with a fertility potential below normal and therefore no false positive results, and 100% sensitivity, and i.e. detects all mammals with a fertility potential below normal and therefore no false negative results. However, due to biological diversity no method can be expected to have 100% sensitive without including a substantial number of false negative results.

The chosen specificity determines the percentage of false positive cases that can be accepted in a given study/population and by a given institution. By decreasing specificity an increase in sensitivity is achieved. One example is a specificity of 95% that will result in a 5% rate of false positive cases. With a given prevalence of 1% of e.g. a fertility potential below normal in a screening population, a 95% specificity means that 5 individuals will undergo further physical examination in order to detect one (1) fertility potential below normal if the sensitivity of the test is 100%.

As will be generally understood by those skilled in the art, methods for screening for fertility potentials are processes of decision making and therefore the chosen specificity and sensitivity depends on what is considered to be the optimal outcome by a given institution/clinical personnel.

In another embodiment said determination of the at least one protein level is performed using a method selected from the group consisting of immunohistochemistry, immunocytochemistry, FACS, ImageStream, Western Blotting, qPCR, RT-PCR, qRT-PCR, ELISA, Luminex, Multiplex, Immunoblotting, TRF-assays, immunochromatographic lateral flow assays, Enzyme Multiplied Immunoassay Techniques, RAST test, Radioimmunoassays, immunofluorescence and immunological dry stick assays. In a more specific embodiment, said determination is performed by immunocytochemistry. In another specific embodiment said determination is performed by ELISA.

In yet an embodiment, said sample is a blood sample, such as blood serum.

Depending on the outcome of the predicition, the subject may be referred to further treatment. Thus, the disclosure further describes the method further comprises referring said subject to assisted fertility treatment, such as mild insemination, in vitro fertilization (IVF), and/or intracytoplasmic sperm injection (ICSI), if said level(s) of OPG, sclerostin, cathepsin K, NFAT, DKK1, DKK2, WNT, Kremen, Frizzled, Parathyroid hormone (PTH) and/or PTH related peptid (PTHrP) is/are equal to or lower than said (first) reference level. In an embodiment, a first reference level may be below 4 pmol/l of OPG in blood serum.

The disclosure further discloses the method further comprises treating said subject with a compound according to the disclosure and/or a composition as according to the disclosure, Denosumab, and/or an antibody being an inhibitor of RANKL, if said level(s) of OPG, sclerostin, cathepsin K, NFAT, DKK1, DKK2, WNT, Kremen, Frizzled, Parathyroid hormone (PTH) and/or PTH related peptid (PTHrP) is/are equal to or higher than said (second) reference level. In an embodiment, a second reference level may be above 3 pmol/l OPG in blood serum, such as in the range 3-5 pmol/l or such as in the range 3-4 pmol/l.

It should be noted that embodiments and features described in the context of one of the aspects or embodiments of the present invention may also apply to the other aspects or embodiments of the invention.

The invention will now be described in further details in the following non-limiting examples.

**Examples**

**Example 1**

This example illustrates the preparation of monoclonal antibodies against RANKL.

Preparations of purified recombinant RANKL, for example, or transfixed cells expressing high levels of RANKL, are employed to generate monoclonal antibodies against RANKL using conventional techniques, such as those disclosed in US Patent 4,411,993. DNA encoding RANKL can also be used as an immunogen, for example, as reviewed by Pardoll and Beckerleg in Immunity 3:165, 1995. Such antibodies are likely to be useful in interfering with RANKL signaling (antagonistic or blocking antibodies).

To immunize rodents, RANKL immunogen is emulsified in an adjuvant (such as complete or incomplete Freund's adjuvant, alum, or another adjuvant, such as Ribi adjuvant R700 (Ribi, Hamilton, MT), and injected in amounts ranging from 10-100 pig subcutaneously into a selected rodent, for example, BALB/c mice or Lewis rats. DNA may be given intradermally (Raz et al., Proc. Natl. Acad. Sci. USA 91:9519, 1994) or intramuscularly (Wang et al., Proc. Natl. Acad. Sci. USA 90:4156, 1993); saline has been found to be a suitable diluent for DNA-based antigens. Ten days to three weeks days later, the immunized animals are boosted with additional immunogen and periodically boosted thereafter on a weekly, biweekly or every third week immunization schedule.

Serum samples are periodically taken by retro-orbital bleeding or tail-tip excision for testing by dot-blot assay (antibody sandwich), ELISA (enzyme-linked immunosorbent assay), immunoprecipitation, or other suitable assays, including FACS analysis.

Following detection of an appropriate antibody titer, positive animals are given an intravenous injection of antigen in saline. Three to four days later, the animals are sacrificed, splenocytes harvested, and fused to a murine myeloma cell line (e.g., NS 1 or preferably Ag 8.653 [ATCC CRL 1580]). Hybridoma cell lines generated by this procedure are plated in multiple microtiter plates in a selective medium (for example, one containing hypoxanthine, aminopterin, and thymidine, or HAT) to inhibit proliferation of non-fused cells, myeloma-myeloma hybrids, and splenocyte-splenocyte hybrids.

Hybridoma clones thus generated can be screened by ELISA for reactivity with RANKL, for example, by adaptations of the techniques disclosed by Engvall et al., Immunochem. 8:871 (1971) and in US Patent 4,703,004. A preferred screening technique is the antibody capture technique described by Beckman et al., J. Immunol. 144:4212 (1990). Positive clones are then injected into the peritoneal cavities of syngeneic rodents to produce ascites containing high concentrations (> 1 mg/ml) of anti-RANK monoclonal antibody. The resulting monoclonal antibody can be purified by ammonium sulfate precipitation followed by gel exclusion chromatography. Alternatively, affinity chromatography based upon binding of antibody to protein A or protein G can also be used, as can affinity chromatography based upon binding to RANKL protein. Using the methods described herein to monitor the activity of the mAbs, both blocking (i.e. antibodies that bind RANKL and inhibit binding to RANK) and non-blocking (i.e. antibodies that bind RANKL and do not inhibit binding) are isolated.

**Example 2**

Introduction

RANKL signalling is important for bone homeostasis, immune system and lactation. RANKL is a transmembrane protein with an extracellular ligand-domain that specifically binds to RANK, a receptor situated in the membrane of the neighboring cell. Binding and activation of RANK induces intracellular signalling often through NFKB activation, which influences cell cycle. Interaction between RANKL and RANK is modulated by OPG secretion from cells in the vicinity as OPG binds to the extracellular domain of RANKL and prevents RANK activation. So the biological effect of RANKL depends on the presence and abundance of RANKL, RANK and OPG. To make things even more complicated, RANKL exists in three different isoforms 1,2,3. One transmembrane form and one cytoplasmic and another secreted form. The different isoforms may not always induce the same effect and the cytoplasmic non-secreted form is in general an inhibitor of RANKL expression. This means that inhibition of RANKL potentially give opposite effects depending on the expression of the specific isoform in the model.

Downstream signalling of RANK is influenced by the presence and activity of TRAF1-6. Depending on which cofactor is present RANK will transmit different signals and thus mediate a different response to the same external stimuli. Besides TRAFs, RANK is also influenced by REL a and b and on the presence of the key downstream signalling mechanism NFKB.

We have previously shown that the Vitamin D receptor (VDR), and all the vitamin D metabolizing enzymes are expressed in the male ejaculatory duct, germ cells and mature spermatozoa. RANKL is a classical vitamin D regulated gene as it contains Vitamin D response elements in the promoter and vitamin D induces transcription of the RANKL gene. RANKL has never, previously, been associated with testicular function and male fertility and has never been shown to be expressed in the gonad. Ablation of RANKL, RANK or OPG in mice does apparently not cause male infertility, but so far there has not been conducted a fertility study or any reports on semen quality.

Denosumab is man-made specific inhibitor of human RANKL signalling as it does not inhibit the murine RANKL or TRAIL signalling. In monkeys a 10-fold higher Denosumab dose than used regularly did not cause any major testicular histological abnormalities or low sperm motility, but fertility testing was not performed. During our work with VDR we found that RANKL was expressed at the RNA level and here we investigated whether RANKL, RANK and OPG are expressed in the gonad of mice and men.

Material and methods

Human tissue samples

Patients were recruited from the andrology clinic at Copenhagen University Hospital (Rigshospitalet), Denmark in accordance with the Helsinki Declaration after approval from the local ethics committee. Adult testis samples were obtained from orchidectomy specimens performed due to TGCT. Tissue surrounding the tumor contained tubules with either CIS cells or normal/impaired spermatogenesis. Each sample was divided into fragments, which were either snap-frozen and stored at -80°C for RNA extraction, or fixed overnight at 4°C in formalin or paraformaldehyde and subsequently embedded in paraffin. An experienced pathologist evaluated all samples and immunohistochemical (IHC) markers were used for TGCT to ensure the histological subtypes of the tumors.

Mouse tissue samples

Leuven Vdr-/- mice and controls were generated and bred using a standard rodent diet (1.0% calcium, 0.7% phosphorus, Sniff R/M-H). All animals were treated according to the National Institutes of Health Guide for the Care and Use of Laboratory Animals, and all experiments were approved by the 'Ethical Committee Animal Tests' of the KU Leuven. Representative groups of mice were killed at 10 or 15 weeks of age, respectively. Mice were exsanguinated under anaesthesia and serum was collected for hormone measurements. Each testis was divided into fragments, which were either snap-frozen and stored at -80°C for RNA or protein extraction, or fixed overnight at 4°C in paraformaldehyde (PFA) or Bouin's solution and embedded in paraffin.

Tissue preparation, quantitative RT-PCR (qRT-PCR) and western blot RNA and cDNA preparation and subsequent qRT-PCR were perfomed. qRT-PCR was performed using specific primers targeting each mRNA. Representative bands from each primer combination were sequenced for verification (Eurofins MWG GmbH, Germany). Changes in gene expression were determined by comparing with RPS20 or B2m ($\beta$2-microglobulin)

Immunohistochemistry (IHC)

All tissue were stained immunohistochemically for RANKL, OPG, and RANK. Briefly, The immunohistochemical (IHC) staining was performed according to a standard indirect peroxidase method and counterstained with Meyer's haematoxylin. All experiments were performed with a negative control staining without the primary antibody. Serial sections were used to examine concomitant expression of RANKL, RANK and OPG, Antigen retrieval was accomplished by microwaving the sections for 15 min in TEG buffer (Tris 6.06 g, EGTA 0.95 g in 5 l, pH 9.0). Sections were washed in 2% hydrogenperoxid to block endogenous peroxidase. Afterwards, all sections were incubated with 2% non-immune goat serum or mouse serum (Zymed Histostain kit, San Francisco,CA, USA) in Tris buffered saline (TBS) to minimize cross-reactivity. Primary antibodies were purchased from Santa Cruz Biotechnology Inc., Santa Cruz, CA. All antibodies were tested by western blotting and initial experiments were carried out with different antibody dilutions (1:50 to 1:500), different buffers (TEG, citrate, Urea) and with different fixatives (formalin, ethanol and none). After 16 h of incubation at 4°C, the sections were incubated with biotinylated goat anti-rabbit IgG (Zymed Histostain kit) or biotinylated donkey antigoat IgG (1:400), before a peroxidase-conjugated streptavidin complex (Zymed Histostain kit) was used as a tertiary layer. Visualization was performed with amino ethyl carbasole (Zymed Histostain kit).The slides were washed with TBS between incubation steps.

Results:

Human

At the RNA level we found a marked expression of RANKL, RANK and OPG in the testis containing normal tubules with complete spermatogenesis (Figure 1). All the isoforms of RANKL was expressed in the testis. Only one isoform was expressed in Sertoli cell only, which differed from the isoform being expressed in the ovary. At the protein level we found expression of RANKL in adult Sertoli cells and in the male germ cells (Figure 3). The expression was cytoplasmic and transmembrane.

The intensity of the staining varied from very low to normal, but RANKL expression was detectable in the vast majority of Sertoli cells and in many germ cells. The presence of RANKL in Sertoli cell only tubules indicates that one isoform is Sertoli specific, while other forms also may be present in germ cells.

Rank was exclusively expressed in the germ cells with a prominent expression in spermatogonia and spermatocytes. Again the expression was mixed cytoplasmic and membranous. OPG was expressed mainly in the peritubular cells and the expression was mainly cytoplasmic. Interestingly the expression of OPG lowered in tissue containing CIS, which may expose the CIS cells to higher RANKL exposure.

Mouse

At the RNA level we found a marked expression of RANKL, RANK and OPG in the testis from both 10 and 15 weeks old animals (Figure 2). All the isoforms of RANKL were expressed in the testis. At the protein level we found expression of RANKL in adult Sertoli cells from two different mice strains. The expression was mixed cytoplasmic and transmembrane. The intensity of the staining varied from very low to normal, but RANKL expression was detectable in the vast majority of Sertoli cells. RANKL appeared to be downregulated in Vdr KO mice, indicating that RANKL also in the testis is regulated by vitamin D. This indicates that the main RANKL isoform present is isoform 1 as RANKL isoform 3 is not regulated by vitamin D in bone and the 10 fold decrease in RANKL following Vdr ablation is substantial.

The specific receptor protein Rank was exclusively expressed in the germ cells with a prominent expression in spermatogonia and spermatocytes. Again the expression was mixed cytoplasmic and membranous. OPG was expressed mainly in the peritubular cells and the expression was mainly cytoplasmic. At the RNA level TRAf1-6 and NFKβ were also expressed in the mouse gonad indicating that the cells are capable of mediating RANK downstream signalling.

Discussion

Here we show that RANKL, RANK and OPG are expressed in the testis. There appears to be an isoform of RANKL exclusively expressed in Sertoli cells, while RANK is expressed in germ cells and OPG is expressed in peritubular cells. This pattern of expression is conserved from mouse to humans, which indicates that RANKL signalling may be involved in spermatogenesis. It is known that signalling from peritubular and Sertoli cells determines germ cell fate and to our knowledge, this is the first signalling involving peritubular, Sertoli and Germ cells.

The RANKL-system is important for bone homeostasis. The factors involved include the transmembrane RANKL protein in osteoblasts that binds and activates a specific receptor RANK in osteoclasts, which induces differentiation of the immature osteoblasts and results in bone resorption. OPG is an endogenous secreted signalling molecule that binds the ligand domain of RANKL and thus inhibits RANK activation and subsequently osteoclast differentiation and activation, which impairs bone resorption. The American pharmaceutical company Amgen developed a synthetic OPG analog (Denosumab) with a long half-life, and EMEA and FDA approved the drug for the treatment of osteoporosis. Denosumab is now used clinically and has very few side effects since RANKL is considered to be expressed only in immune cells, teeth and breast besides bone. FDA or EMEA did not request a male fertility study before the drug could be used clinically because patients with osteoporosis in general are females and of old age.

We have new data showing that one isoform of RANKL is expressed in the male gonad but not in the female counterpart. This RANKL isoform appears to be expressed in Sertoli cells, while the specific receptor RANK is expressed in spermatogonia, and OPG is expressed in peritubular cells. Expression of these proteins is found in both mice and men, and RANKL and RANK are also expressed in Sertoli and germ cells in dysgenetic tubules and pre-cancerous testicular cells (CIS), while CIS tubules have low OPG expression. All the downstream mediators of RANK signalling (TRAF1-6 and NFKβ) are also expressed in the testis from rodents and men, and NFKβ is known to be important for the proliferation-apoptotic switch in the male gonad. Rankl signalling may thus be an important regulator of germ cell proliferation. Inhibition of RANKL in the normal testis may mainly be the Sertoli specific form as it is approachable on the proximal site of the blood-testis barrier, while the presumed cytoplasmic form being expressed in the later stages of spermatogenesis probably is inaccessible for circulating Denosumab. The effect of Denosumab cannot be adapted directly from experiments using OPG treatment as Amgen suggested that Denosumab unlike OPG does not block TRAIL, which is important for male germ cell apoptosis but only RANKL.

In mice, the expression of RANKL, RANK and OPG is conserved and lessons from Vdr KO mice showed that testicular RANKL is regulated by vitamin D. The RANKL gene has two VDRE and the marked downregulation in Vdr KO mice support the fact that downregulation of RANKL is unlikely to cause a massive decrease in spermatogenesis since these mice have normal testicular histology although they have a modestly impaired fertility, which may be due to RANKL or more likely other causes (see Blomberg Jensen M et al. Characterization of the testicular, epididymal and endocrine phenotypes in the Leuven Vdr-deficient mouse model: targeting estrogen signalling, Mol Cell Endocrinol. 2013 Jul 11;377(1-2): 93-102). Moreover, In bone RANKL isoform 1 is regulated by vitamin D, while isoform 3 is not, and this indicates that the massive downregulation in Vdr mutants indicates that RANKL isoform 1 is the main isoform present.

In conclusion, The presence of RANKL, RANK and OPG in the gonad has never been shown before, and their expression indicates that RANKL signalling through NFKβ may be involved in regulation of proliferation of germ cells. This implies that regulation of the RANKL system may be clinically relevant as it may lower or increase the production of spermatozoa.

**Example 3**

Introduction

RANKL signalling is important for bone homeostasis, immune system and lactation. RANKL is a transmembrane protein with an extracellular ligand-domain that specifically binds to RANK, a receptor situated in the membrane of the neighboring cell. Binding and activation of RANK induces intracellular signalling often through NFKB activation, which influences cell cycle. Interaction between RANKL and RANK is modulated by OPG secretion from cells in the vicinity as OPG binds to the extracellular domain of RANKL and prevents RANK activation. So the biological effect of RANKL depends on the presence and abundance of RANKL, RANK and OPG. To make things even more complicated, RANKL exists in three different isoforms 1,2,3. One transmembrane form and one cytoplasmic and another secreted form. The different isoforms may not always induce the same effect and the cytoplasmic non-secreted form is in general an inhibitor of RANKL expression. This means that inhibition of RANKL potentially give opposite effects depending on the expression of the specific isoform in the model. The two cell lines TCAM2 and NTERA2 express two different isoforms of RANKL and modulation of this signaling in these germ cell derived cells may indicate the effect of RANKL in the testis.

Denosumab is a man-made specific inhibitor of human RANKL signalling as it does not inhibit the murine RANKL or TRAIL signalling. In monkeys a 10-fold higher Denosumab dose than used regularly did not cause any major testicular histological abnormalities or low sperm motility, but fertility testing was not performed.

Materials and methods

Human Cell lines

The EC-derived cell line NTera2 that retain less germ cell characteristics and the seminoma-derived TCam-2 cell line, which retain germ cell characteristics were used in this study. Cells were grown under standard conditions at 37°C in 5% $CO_2$ atmosphere in DMEM (NTera2) or RPMI 1640 (TCam-2) supplemented with glutamine (58.5 mg/ml), penicillin (100 U/ml) and streptomycin (100 mg/ml) (all Gibco). To investigate the effect on proliferation, RANKL (100 ng/ml), CSF-1 (100 ng/ml), Denosumab (100 ng/ml) were added alone or in combination to the media. DMSO was used as solvent, and all control samples were DMSO treated. Cells were plated in 25-$cm^2$ flasks and detached using 0.05% trypsin-EDTA for 5 min at 37°C. One part was centrifuged down and the cell pellet was snap-frozen for RNA purification and cDNA synthesis for RT-PCR. qRT-PCR was performed using specific primers targeting each mRNA. Representative bands from each primer combination were sequenced for verification (Eurofins MWG GmbH, Germany).

Survival assay

The survival assay determines the metabolic activity of living cells by measuring the reduction of the oxidized form of resazurin and the concomitant increase in the fluorescent intermediate. Cells were counted manually in a Bürker-Türk counting chamber (Tiefe 0.100 mm, 0.0025 $mm^2$) and $5 \times 10^3$ cells/well were seeded into wells of 96-well plates and incubated overnight before treatment. For co-treatment experiments $1 \times 10^3$ cells/well were plated in 96-well plates, incubated overnight before initial treatment with 100 ng/ml of the selected compound for 20 hours. Negative controls (media only) and positive controls ($1 \times 10^3$ cells in normal media) were included on all plates. Survival of cells was determined using the TOX-8 *in vitro* toxicity assay (Sigma Aldrich) according to manufacturer's instructions. At the end of treatment resazurin dye from the assay was added to the media followed by 4-6 hours incubation. Plates were then measured on an ELISA microplate reader (Tecan, Sunrise). To calculate percent survival, raw absorbance values were subtracted values from reference wavelength, and the blank samples (media only). Resulting values were averaged (n=8 for each treatment) to generate a final absorbance value. Untreated cells were considered as 100% survival and used to calculate the percent survival of treated cells. Each experiment was repeated at least three times in independent experiments.

Results:

NTera2 cells express one isoform of RANKL that differs from TCAM2 cells. The isoform expressed in the TCAM2 cells is similar to the Sertoli cell specific isoform in Sertoli cell only (SCO), which differs from the one being expressed in the ovary. This isoform may be expressed in the Sertoli cells of normal testis, however, one cannot exclude that normal and SCO only Sertoli cells have different RANKL isoforms. RANK and the downstream signaling machinery are expressed in both cell lines, which mean that they will both be responsive to RANKL treatment (Figure 1). OPG is exclusively expressed in NTera2 cell, which means that they have a natural inhibitor for RANKL and may adapt to increased exposure and be less sensitive, while TCAM2 does not express OPG.

In NTERA2 cells (Figure 4) we find an aberrant response since both Denosumab and RANKL treatment induce proliferation of the cells after 4 and 20 hours. Denosumab has the strongest effect, but it is not clear why both compounds induce proliferation. One possibility is that Denosumab blocks a specific transmenbrane isoform of RANKL on the NTera2cells, which thereby induces proliferation. While exogenous RANKL stimulates RANK as a cytoplasmic isoform and thereby occupies the receptor, which prevents the endogenous transmembrane RANKL to activate RANK on the neighbor cell. The response of the exogenous RANKL may therefore mimic an effect of secreted isoform, which therefore is antagonistic and induces proliferation. The production of OPG may also influence the data, as it may inhibit the endogenous RANKL and this inhibition may be lowered when exogenous RANKL is added.

In TCAM2 cells (Figure 5) another isoform is expressed and here RANKL treatment decreases proliferation as expected, while Denosumab again stimulates proliferation although only modestly after 4 hours. All the treatments comprising RANKL lowers proliferation significantly after 20 hours, while Denosumab shows insignificant tendencies towards higher proliferation after 20 hours, but it is significant after 4 hours. Another reason to the difference is that, TCAM2 cells does not express OPG and can therefore not abrogate the effect of exogenous RANKL treatment, which may influence their high sensitivity to the treatment.

Discussion

These data show clearly that modulation of the RANKL pathway influence cell survival of cancer cells derived from germ cell tumors. Both RANKL and Denosumab treatment induces marked changes in cell survival. some of the differences in the observed response may be due to the different RANKL isoforms in the two cells. Although the presence of OPG also differs, which obviously influence the results. The changes in germ cell proliferation are induced extremely fast as expected and support the fact that both RANKL and Denosumab can induce a potent and rapid effect on germ cell survival.

The results from the hanging drop culture of CIS and normal germ cells clearly demonstrate that RANKL signaling between Sertoli and Germ cells is important for germ cell survival and proliferation. RANKL lowers proliferation by inducing apoptosis, while Denosumab has the opposite effect. These results may partly be extrapolated to the normal testis, but spermatogonia do not have a strong RANKL expression as most CIS cells, which may influence the results in the normal germ cells. However, the results show clearly that Denosumab and RANKL treatment of germ cell in the normal somatic environment regulate germ cell proliferation.

In conclusion, by using two different models with different RANKL isoforms, presence or absence of endogenous OPG, different cell lines we have shown that Denosumab regulates germ cell proliferation.

**Example 4**

Ex vivo treatment of human testis with denosumab and RANKL.

Introduction

RANKL signalling is important for bone homeostasis, immune system and lactation. RANKL is a transmembrane protein with an extracellular ligand-domain that specifically binds to RANK, a receptor situated in the membrane of the neighbouring cell. Binding and activation of RANK induces intracellular signalling often through NFKB activation, which influences cell cycle. Interaction between RANKL and RANK is modulated by OPG secretion from cells in the vicinity as OPG binds to the extracellular domain of RANKL and prevents RANK activation. So the biological effect of RANKL depends on the presence and abundance of RANKL, RANK and OPG. To make things even more complicated, RANKL exists in three different isoforms 1, 2, and 3. One transmembrane form and one cytoplasmic and another secreted form. The different isoforms may not always induce the same effect and the cytoplasmic non-secreted form is in general an inhibitor of RANKL expression. This means that inhibition of RANKL potentially give opposite effects depending on the expression of the specific isoform in the model. We have found that RANK is exclusively expressed in germ cells, which therefore are the only cells able to respond to RANKL and Denosumab treatment directly. RANKL is expressed in both Sertoli and Germ cells, while OPG is expressed in peritubular cells. We have recently established an organ culture model in which small tissue fragments are cultured in 'hanging drops' of optimised media. This culture approach is applicable for human testis tissue, tissue containing CIS and testicular cancer tumours obtained from orchidectomy specimens of patients with testicular cancer. We have successfully cultured normal testis, tissue with CIS and TGCT for up to 2 weeks without any observed negative changes. This organ culture model allows studies on the mechanisms of RANKL signalling pathways in a dynamic and functional manner, and since the model preserves the integrity of the tissue it has the advantage that the germ cells are sustained within their somatic niche (Sertoli and Peritubular cells) allowing continuous interactions with the somatic cells during treatment experiments. Denosumab is man-made specific inhibitor of human RANKL signalling as it does not inhibit the murine RANKL or TRAIL signalling as the endogenous produced OPG does. In monkeys a 10-fold higher Denosumab dose than used regularly did not cause any major testicular histological abnormalities or low sperm motility, but fertility testing was not performed.

*Materials and methods*

Human tissue sample collection and preparation

Patients were recruited from the Andrology Clinic of the Department of Growth and Reproduction at Copenhagen University Hospital (Denmark) in accordance with the Helsinki Declaration, and following approval from the local ethics committee (permit nr. H-1-2012-007). All participants gave informed consent before orchidectomy for treatment of testicular cancer. The orchidectomy specimens were transported immediately after surgical removal to the Pathology Department and were divided into tumour and macroscopically normal areas. The majority of the tissue was assigned for diagnostic analysis, with the remainder for research. The sample portions assigned for research were placed immediately in media (see below) and transported to the laboratory. Within 2h of surgical removal, the specimens were cut into ~1mm3 pieces (an average seminiferous tubule is 150$\mu$m in diameter) and placed into hanging drop cultures in media containing DMEM F12, penicillin (100U/ml-1), streptomycin (100mg/ml-1), insulin, transferrin and selenium ($\times$ 1) with either 10%fetal bovine serum (FBS) or 0.1% bovine serum albumin (BSA). In the initial phase of culture condition optimisation, several different basic media were tested, including DMEM, RPMI-1640 and MEM$\alpha$, but no apparent differences in survival or tissue morphology were observed (data not shown). All cell media and supplements were from Gibco (Naerum, Denmark), except BSA (Sigma-Aldrich, Broendby, Denmark). To set-up the cultures, 30 $\mu$l drops of medium were prepared on the lid of a Petri-dish (NUNC cell culture Petri-dish). Individual tissue pieces were placed into each drop, and then the dish was carefully inverted to keep the drops intact with the tissue suspended. DPBS (10ml) was added to the bottom of the dish to prevent dehydration. Tissues were cultured at 34°C in 5% CO2 for up to 14 days, with media changed every 48h. The experimental set-up included at least three replicates of tissue pieces cut from the same original tissue piece for each of the following: fixation, RNA purification and survival assay. When the culture period was completed, samples were collected into 4% paraformaldehyde (PFA) fixative (30min at room temperature, 4°C overnight) followed by paraffin embedding for histological analysis and immunohistochemistry, placed in RNAlater (Ambion, Austin, TX, USA) for RNA purification and real-time PCR analyses or set-up in a survival assay.

Survival assay

To evaluate whether the tissue pieces contained living cells, a survival assay was used to determine total metabolic activity using the TOX-8 in vitro toxicity assay (Sigma-Aldrich), according to the manufacturer's instructions. In brief, tissue pieces were transferred to a 96-well dish with 90$\mu$l media and 10$\mu$l resazurin dye (Sigma-Aldrich) and incubated for 5h at 34°C together with negative experimental controls (media+resazurin dye, without tissue) that were included on all plates. The reduction of the oxidised form of resazurin (blue) and the concomitant increase in the fluorescent intermediate (red) were then measured on an ELISA microplate reader (Tecan Sunrise, Männedorf, Switzerland) according to the manufacturer's instructions (Sigma-Aldrich).

Immunohistochemistry

In brief, paraffin sections were deparaffinised and rehydrated. Antigen retrieval was accomplished by microwaving the sections for 15min in retrieval buffer. Sections were then incubated with 2% non-immune goat serum (Zymed Histostain kit, San Francisco, CA, USA) or 0.5% milk powder diluted in Tris buffered saline (TBS) to minimise cross-reactivity. Primary antibodies, dilutions and retrieval buffers. After 16 h of incubation at 4°C and 1h at room temperature, the sections were incubated with biotinylated goat anti-rabbit IgG (Zymed Histostain kit) or biotinylated goat anti-mouse IgG (1:400), before a peroxidase-conjugated streptavidin complex (Zymed Histostain kit) was used as a tertiary layer. Visualisation was performed with amino ethyl carbasole (AEC) (Zymed Histostain kit) yielding a deep red colour. Between incubation steps, the slides were washed with TBS. For negative controls, serial sections were processed, with the primary antibody replaced by the dilution buffer alone. None of the control slides showed any staining. Counterstaining was performed with Mayer's haematoxylin. Serial sections were used to test for expression of PLAP (signal detected in CIS and seminoma cells), KI-67, BrdU incorporation, cleaved caspase, KIT and AP2y. Two independent investigators evaluated all staining. All sections were investigated manually on a Nikon Microphot-FXA microscope (Tokyo, Japan) and were then scanned on a NanoZoomer 2.0 HT (Hamamatsu Photonics, Herrsching am Ammersee, Germany) and analysed using the software NDPview version 1.2.36 (Hamamatsu Photonics). To evaluate the immunohistochemical (IHC) stainings in a more quantitative manner, the percentages of KI-67-, BrdU-, KIT- and AP2y-stained cells were determined by cell counting. The proliferation markers KI-67 and BrdU were expressed only in normal and malignant germ cells, with a weakly KI-67-stained Sertoli cell observed only very rarely. In normal testis samples with complete spermatogenesis, we counted only spermatogonia and spermatocytes; in samples containing CIS, we determined also the proportion of stained CIS cells. For all quantifications (KI-67, BrdU, KIT and AP2y), a minimum 3.5 mm2 tissue were evaluated by two different investigators in each of three tissue fragments per treatment from each patient. Tissue pieces from at least three different patients were included for vehicle, rankl and denosumab treatment. Both positive and negative cells were counted to calculate the percentage of stained cells.

Proliferation assay

A BrdU incorporation assay was used to determine the presence of proliferating germ cells just before the end of the culture period. BrdU labelling reagent (Invitrogen, Camarillo, CA, USA) was diluted 1:100 in media and tissue pieces were set up as hanging drop cultures in BrdU containing media for 3h. Tissue pieces were then washed twice in PBS for 5min followed by fixation and paraffin embedding as described above. BrdU was visualised by immunohistochemistry using a BrdU antibody as described in the Immunohistochemistry section, and positively stained cells were considered as proliferating.

**Results**

The overall morphology of tissue with areas containing full spermatogenesis termed normal testis tissue (NT) and testis tissue with tubules containing CIS cells was maintained for up to 14 days in hanging drop tissue cultures. Staining of NT tissue with Haematoxylin & Eosin (HE) demonstrated the presence of all germ cell types, and staining with the CIS marker PLAP confirmed whether CIS cells were present. After 7-10 days of culture, a loss of more differentiated germ cells, particularly the post-meiotic spermatids, was observed in tissue from some patients, but this was not a consistent finding. In tissue containing tubules with CIS cells, staining with HE and PLAP demonstrated that the tubules contained predominantly CIS cells (positive for PLAP) and Sertoli cells and that morphology of the tissue was preserved during culture for up to 14 days.

Two measures of cell proliferation were applied in this study: IHC staining with KI-67 and a BrdU incorporation assay. To directly compare the proliferation measures, KI-67 and BrdU staining's were performed on serial sections of tissue pieces. In general, a significantly ($P<0.05$) higher number of labelled cells was found by detection of KI-67 compared with BrdU. These data also indicate that BrdU incorporation, followed by antibody detection, is a more specific marker for cell proliferation than the KI-67 staining. This is expected, as BrdU is incorporated into DNA as a thymidine analogue only during S phase of the cell cycle in the last 3h of the culture period. In contrast, KI-67 is expressed in all phases of the cell cycle, except during G0/G1 arrest, and the following results will only be presented with BrdU incorporation.

Results from treatment of human testis specimens in hanging drop culture revealed a marked change in germ cell proliferation. In total, we used testis from four men, but only three of the men had testicular tissue present in the specimens. The size of each specimen used for each treatment was on average 5,92mm2. Each treatment lasted for 48 hours and we used two control specimens from all testis to avoid bias from different histology in the serial sections. Using this approach, we ensure that the germ cells are kept in their normal intratubular niche in contact with the Sertoli cells. This means that the cells are exposed to the Sertoli specific form of RANKL. Two specimens contained both normal tissue and premalignant CIS cells, while one did not contain normal spermatogenesis but the only germ cells found were CIS cells. Treatment with Denosumab and OPG increased proliferation of germ cells markedly, while RANKL and other drugs known to influence RANKL signaling in bone CSF-1 and PTHrP do not have a similar effect treatment was unable to induce a significant change in germ cell proliferation as assessed by BrdU incorporation (Figure 6). Denosumab induced an increase in the fraction of proliferation germ cells by more than 70% in two of the patients, while Denosumab induced a 11-fold increase in the last patient who had a very severe testicular phenotype histologically. The positive effect of Denosumab was comparable with the effect of OPG-FC which besides inhibiting RANKL also blocks TRAIL.

CIS cells resemble fetal germ cells and differ from spermatogonia because some express RANKL themselves. However, they are situated in the correct niche as spermatogonia and respond in a similar manner as the normal germ cells since RANKL decreases proliferation by inducing Caspase dependent apoptosis, while Denosumab increases proliferation by inhibiting Caspase dependent apoptosis (Figure 7). The effect is rapid and marked and the opposite effect of exogenous RANKL treatment is a strong indicator of a reproducible effect of treatment in vivo because the cells are exposed in their natural environment.

**Discussion**

These data show clearly that modulation of the RANKL pathway influence germ cell survival and proliferation. Both RANKL and Denosumab treatment induces marked changes in cell survival. Some of the differences in the observed response may be due to the different RANKL isoforms present in the testis. The changes in germ cell proliferation are induced extremely fast as expected and support the fact that both RANKL and Denosumab can induce a potent and rapid effect on germ cell survival.

The results from the hanging drop culture of CIS and normal germ cells clearly demonstrate that RANKL signaling between Sertoli and Germ cells is important for germ cell survival and proliferation. The results show clearly that Denosumab and RANKL treatment of germ cell in the normal somatic environment regulate germ cell proliferation, while other drugs known to influence RANKL signaling in bone CSF-1 and PTHrP do not have a similar effect (Figure 6).

In conclusion, by using germ cells situated in their normal environmental niece, we have shown that Denosumab and OPG induce germ cell proliferation.

**Example 5**

In vivo effects of inhibition of testicular RANKL inhibition by infusing human OPG-FC, RANKL, as well as vehicle to mice for a period of 14 days.

Mice are recognized models for the study of pharmacodynamics and pharmacokinetic effects of medical treatment. Unfortunately, we were unable to test the effect of Denosumab because Denosumab is unable to block mouse RANKL. Instead we used human OPG, which effectively block the effect of mouse RANKL and may act in a similar fashion as Denosumab as previously shown in example 4.

**Material and methods**

Animals

C57BL/6J mice, 15 males, 7-8 weeks of age from Taconic Europe A/S. The mice were caged in European standard cages type II. Bedding Jeluxyl HW 300/500 (Jelu, J.Ehrler GmbH & Co KG, Ludwigsmühle, D-73494 Rosenberg, Germany). The bedding was changed once a week in a laminar flow unit. The air was exchanged approximately 12 times per hour in the stable. Temperature was 20°C to 24°C, and controlled via the ambient ventilation system. Light cycle was 12-hour dark and 12-hour light (lights on 06.00). Diet Altromin 1324, produced by Altromin, Im Seelenkamp 20, 32791 Lage, Germany. Water was UV-sterilized and water bottles was refilled when necessary during acclimatization and experiment.

Diet and was administered ad libitum.

The following test articles were used

| Gr. No. | Test article 1 (ID) | Amount, received | Storage | Special |
|---------|---------------------|------------------|---------|---------|
| 1 | OPG-FC (cyt-177-c) | 2 mg | -20C | Powder(compound) |
| 2 | RANKL (cyt-692-b) | 200μl (20 μg/ 40 μl) | -20C | Liquid |
| 3 | Vehicle (PBS) | - | Room temp | Liquid |

The following dose solutions were used:

| Gr. No. | Test article 1 (ID) | Conc | Dose Vol | Dose | Route |
|---------|---------------------|------|----------|------|-------|
| 1 | OPG-FC (cyt-177-c) | 0,2 mg/ml | 5 ml/kg | 1 mg/kg | i.p |
| 2 | RANKL (cyt-692-b) | 8 μg/ml | 5 ml/kg | 40 μg/kg | i.p |
| 3 | Vehicle | - | 0,1 ml/mice | - | i.p |

Experimental design

Fifteen (15) mice (Gr. 1-3) was randomized into 3 groups of 5 animals. Treatment of the mice take place as indicated in the table below. The mice was monitored for abnormal clinical symptoms. Body weights will be determined before each round of dosing. The mice was terminated after 14 days of dosing. At termination the mice was euthanized by cervical dislocation. Three hundred (300) μl blood was collected from each animal and 100-150 μl serum will be prepared. Kidneys and testicles will be dissected free and weighed. The samples were preserved as follows: Kidney samples: One Snap frozen and one in buffered formalin or PFA Testis samples: One testis in formalin or PFA, ½ testis in bouins fixative and ½ testis snap frozen. Epididymis snap frozen.

| Gr. No. | n | Test article 1 (ID) | Dose | Route | Frequency | Special |
|---------|---|---------------------|------|-------|-----------|---------|
| 1 | 5 | OPG-FC (cyt-177-c) | 1 mg/kg | i.p | 2 x weekly | |
| 2 | 5 | RANKL (cyt-692-b) | 40 μg/kg | i.p | 3 x weekly | |
| 3 | 5 | Vehicle | - | i.p | 3 x weekly | |

Clinical observations

All animals are inspected on a daily basis for their general condition. Any clinical signs or behavioral abnormalities are recorded. Caudal epididymides were minced in 1 ml PBS 1X and the number of cells released counted after 1 hour. The total sperm count was assessed in the final suspension by using a hemocytometer.

Humane end points and premature termination

Any animals showing clinical signs of more than mild pain or moderate distress will be humanely euthanized. This includes animals exhibiting clinical signs that exceed the limits of the humane end points. If an animal is found dead or is euthanized prematurely during the study then the time of death will be recorded as precisely as possible. The body weight will be measured before euthanasia or at the time of finding of the dead animal. If possible, a post mortem examination will be conducted on the animal. The animal external and internal appearance and the pivotal organs will be inspected and described in accordance to the 'Post Mortem Examination Schedule' and the organ weights will be recorded. The pivotal organs will be preserved in formalin in one vial per animal. At the termination of the control group animals then identical post mortem examinations will be conducted on the control group animals. This is to make further investigation possible.

**Results**

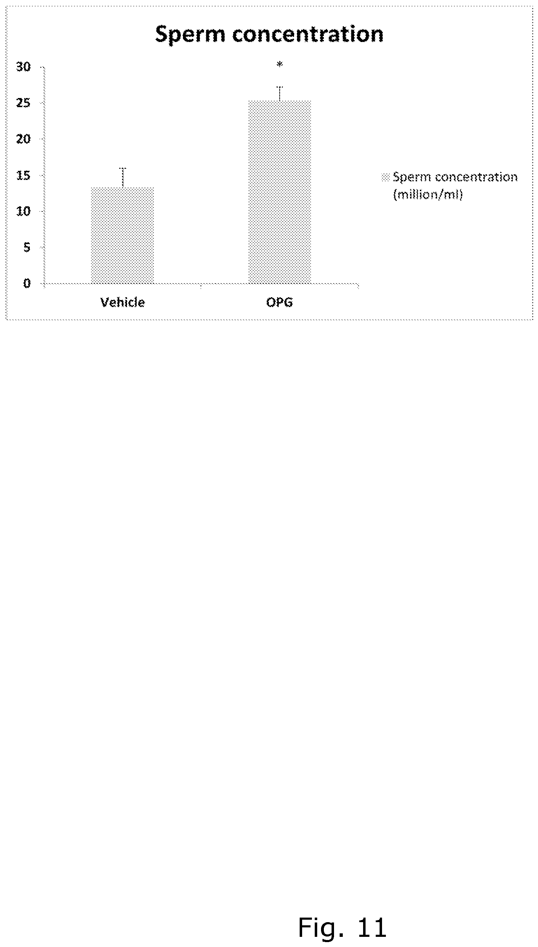

There was no major difference in body weight following treatment with Vehicle, RANKL or OPG. The animals showed no sign of distress. A comprehensive evaluation of male fertility potential was not performed, but the weight of the reproductive organs (testis and epididymis) was significantly higher in OPG treated mice compared with vehicle and RANKL treated mice respectively (Figures 8 and 9). In addition, more tubules appeared wider in OPG treated mice compared with RANKL and vehicle treated 10 weeks old mice (figure 10, top), and, germ cell epithelial width was significantly wider independent of the stage of spermatogenesis in the OPG treated mice (Figure 10, buttom). The increased germ cells in the OPG treated mice may explain the difference in epididymal weight, which could be caused by a higher number of accumulating spermatozoa in the OPG treated mice compared with RANKL treated mice. To verify this we released spermtatozoa from cauda epididymis from both vehicle and OPG treated mice since these spermatozoa are fully matured and ready for ejaculation and therefore is an indirect marker of their fertility potential. We found a marked difference in sperm concentration. OPG treated mice had an significant higher (p=0.02) average sperm concentration of 25. 3 million per ml compared with vehicle treated mice 13.4 million per ml (Figure 11). The marked difference in sperm count between OPG and vehicle treated mice is in line with the significant differences in the organg weight of testis and epididymis and the width of the germ cell layer in the testis, which strongly support that OPG treated mice have a better semen quality and as a result better fertility potential.

**Discussion**

In this comprehensive study of the reproductive organs of mice treated with exogenous RANKL, OPG or vehicle for two weeks, we showed that inhibition of RANKL signaling by using OPG treatment lead to significant changes in the weight and histology of the testis and epididymis. This was not a surprise because RANKL is expressed in the testis and intro and ex vivo data have showed that inhibition of RANKL with Denosumab and OPG results in germ cell proliferation. In accordance, the germ cell layer in OPG treated mice is significantly wider compared with vehicle and RANKL treated mice indicating that the increase in testis weight is due to increased number of germ cells. Especially epidididymis weight is significantly different between RANKL and OPG treated mice, which may be due to lower sperm number situated in the epididymis of RANKL treated mice compared with OPG treated mice. Actually, we were able to show that sperm counts were significantly higher in OPG treated mice compared with vehicle treated mice, which strongly support that OPG treatment improves fertility potential in mice. Importantly, exogenous treatment with OPG and RANKL will not only influence testicular signaling as RANKL and RANK also is expressed in the skeleton and the immune system, which indirectly also could influence testicular function. This may explain the increase in testis weight following RANKL treatment, which like the in vitro experiments again may be due to difference in function of the transmembrane and secreted form of RANKL.

In conclusion, The marked difference in sperm count between OPG and vehicle treated mice is in line with the significant differences in the organ weight of testis and epididymis and the width of the germ cell layer in the testis, which strongly support that OPG treated mice have a better semen quality and as a result better fertility potential.We provide evidence that the presence of RANKL, RANK and OPG in murine testis may be of clinical importance and exogenous treatment with OPG may be used as a fertility improving drug

**Example 6**

We have now shown that RANKL signalling is important not only for for bone homeostasis but also reproductive function. RANKL is a transmembrane protein with an extracellular ligand-domain that specifically binds to RANK, a receptor situated in the membrane of the neighboring cell. Binding and activation of RANK induces intracellular signalling often through NFKB activation, which influences cell cycle. Interaction between RANKL and RANK is modulated by OPG secretion from cells in the vicinity as OPG binds to the extracellular domain of RANKL and prevents RANK activation. So the biological effect of RANKL depends on the presence and abundance of RANKL, RANK and OPG. Downstream signalling of RANK is influenced by the presence and activity of TRAF1-6. Depending on which cofactor is present RANK will transmit different signals and thus mediate a different response to the same external stimuli. Besides TRAFs, RANK is also influenced by REL a and b and on the presence of the key downstream signalling mechanism NFKB. OPG blocks RANKL signalling but is also able to inhibit TRAIL signalling indicating that at least part of the response of OPG could be through TRAIL and not only RANKL signalling. Denosumab is man-made specific inhibitor of human RANKL signalling as it does not inhibit the murine RANKL or TRAIL signalling.

The marked induction of germ cell proliferation and sperm number following treatment with Denusomab and OPG indicates that other factors influencing the RANKL system also may exert an influence on germ cell proliferation if they are present in the testis. RANKL is exclusivelsy expressed in the Skelelon immune cells and lactating breast and severeal other regulators of RANKL signalling and osteocalst function are thought to be exclusively expressed in the bone. We suggest that these potent regulators of RANKL signalling and ostecoclast function and activity also may be expressed in the testis and that their function is the testis is to regulate RANKL signalling and germ cell proliferation. Local paracrine regulators of RANKL thought to be in the testis is Sclerostin, Cathepsin K, DKK1, DKK2, Frizzled, Wnt's, NFATs etc. Presence of these factors in the testis would corroborate the importance of RANKL signalling in the gonad. It would also have clinical implications since several pharmaceutical companies currently are testing specific antibodies or inhibitors against Sclerostin, Cathepsin K, DKK1 to treat osteoporosis, and presence of these factors in the testis will therefore indicate a yet unrecognised reproductive effect.

*Human tissue samples*

Patients were recruited from the andrology clinic at Copenhagen University Hospital (Rigshospitalet), Denmark in accordance with the Helsinki Declaration after approval from the local ethics committee. Adult testis samples were obtained from orchidectomy specimens performed due to TGCT. Tissue surrounding the tumor contained tubules with either CIS cells or normal/impaired spermatogenesis. Each sample was divided into fragments, which were either snap-frozen and stored at -80°C for RNA extraction, or fixed overnight at 4°C in formalin or paraformaldehyde and subsequently embedded in paraffin. An experienced pathologist evaluated all samples and immunohistochemical (IHC) markers were used for TGCT to ensure the histological subtypes of the tumors.

*Mouse tissue samples*

Leuven Vdr-/- mice and controls were generated and bred using a standard rodent diet (1.0% calcium, 0.7% phosphorus, Sniff R/M-H). All animals were treated according to the National Institutes of Health Guide for the Care and Use of Laboratory Animals, and all experiments were approved by the 'Ethical Committee Animal Tests' of the KU Leuven. Representative groups of mice were killed at 10 or 15 weeks of age, respectively. Mice were exsanguinated under anaesthesia and serum was collected for hormone measurements. Each testis was divided into fragments, which were either snap-frozen and stored at -80°C for RNA or protein extraction, or fixed overnight at 4°C in paraformaldehyde (PFA) or Bouin's solution and embedded in paraffin.

Tissue preparation, quantitative RT-PCR (qRT-PCR) and western blot RNA and cDNA preparation and subsequent qRT-PCR were perfomed. qRT-PCR was performed using specific primers targeting each mRNA . Representative bands from each primer combination were sequenced for verification (Eurofins MWG GmbH, Germany). Changes in gene expression were determined by comparing with RPS20 or B2m ($\beta$2-microglobulin)

*Immunohistochemistry (IHC) and Western blot*

All tissue were stained immunohistochemically for Sclerostin and Cathepsin K. Briefly, The immunohistochemical (IHC) staining was performed according to a standard indirect peroxidase method and counterstained with Meyer's haematoxylin. All experiments were performed with a negative control staining without the primary antibody. Serial sections were used to examine concomitant expression of antibodies, Antigen retrieval was accomplished by microwaving the sections for 15 min in TEG buffer Tris 6.06 g, EGTA 0.95 g in 5 l, pH 9.0). Sections were washed in 2% hydrogenperoxid to block endogenous peroxidase. Afterwards, all sections were incubated with 2% non-immune goat serum or mouse serum (Zymed Histostain kit, San Francisco,CA, USA) in Tris buffered saline (TBS) to minimize cross-reactivity. Primary antibodies were purchased from Santa Cruz Biotechnology Inc., Santa Cruz, CA. All antibodies were tested by western blotting and initial experiments were carried out with different antibody dilutions (1:50 to1:500), different buffers (TEG, citrate, Urea) and with different fixatives (formalin, ethanol and none). After 16 h of incubation at 4°C, the sections were incubated with biotinylated goat anti-rabbit IgG (Zymed Histostain kit) or biotinylated donkey antigoat IgG (1:400), before a peroxidase-conjugated streptavidin complex (Zymed Histostain kit) was used as a tertiary layer. Visualization was performed with amino ethyl carbasole (Zymed Histostain kit).The slides were washed with TBS between incubation steps. Frozen tissues prepared for western blotting (WB) included normal adult testis, testis with CIS as well as homogeneous seminoma and embryonal carcinoma samples. WB was carried out with HRP-conjugated secondary antibodies followed by chemiluminescent detection with Pierce ECL detection (No. 34095). Primary antibodies were used in dilution 1:200. Protein was loaded in each lane. $\beta$-Actin antibody was used as loading control. Secondary antibodies were HRP-conjugated rabbit anti-mouse (Dako, P0260) and HRP-conjugated swine anti-rabbit (Dako, P0217), both diluted 1:1000.

**Results**

We found a marked expression of most bone markers investigated at at mRNA level in testis from both mouse and human (Figure 12 and 13) Interestingly, the expression of some of the Bone factors differed between normal and dysgenetic testicular human samples in humans. For instance DKK1. The expression of DKK1 appears to be lower in normal testis compared with embryonal carcinoma and cell lines. This may be of clinical interest bacuse the growth pattern differs between the different tissues investigated. Interestingly, we also found a marked expression of all investigated bone signals in the testis from both wildtype VDR and Vdr KO mice. Again we found a difference in the expression of DKK1 (Figure 13), which resemble the expression profile of RANKL and SOST because they were also low in VDR KO mice. Currently we are investigating the protein expression of all the bone signals. In Figure 14 we show by using two different antibodies identival expression with both of them because sclerostin is expressed in peritubular and germ cells. The data is supported by WB confirming the presence of the protein and by qPCR showing that SOST is abundantly expressed but decreases in VDR ko mice. Cathepsin K protein was also present in mainly peritubular but also some germ cells, which was supported by western blot. Another potent regulator of RANKL is PTH and PTHrP, and we found expression of PTHrP and the PTHR in the testis as shown in figure 16. All these data corroborate the presence of all these bone signals and highlights a role of these bone factors in the testis because the proteins are expressed. Moreover, all the factors are expressed in close vicinity of each other, which makes sense because they are interconnected. They are also in close proximity with the testicular RANKL system which support their regulatory role and influence in the testis as it has been shown in the skeleton. The fact that any modification is of importance is supported by the finding that OPG treated mice had an significant higher (p=0.02) average sperm concentration of 25. 3 million per ml compared with vehicle treated mice 13.4 million per ml. We suggest that inducing differences in sclerostin, cathepsin K, DKK1 etc in the testis, may change the testicular expression level of RANKL, RANKL or OPG and thereby induce secondary changes in sperm output

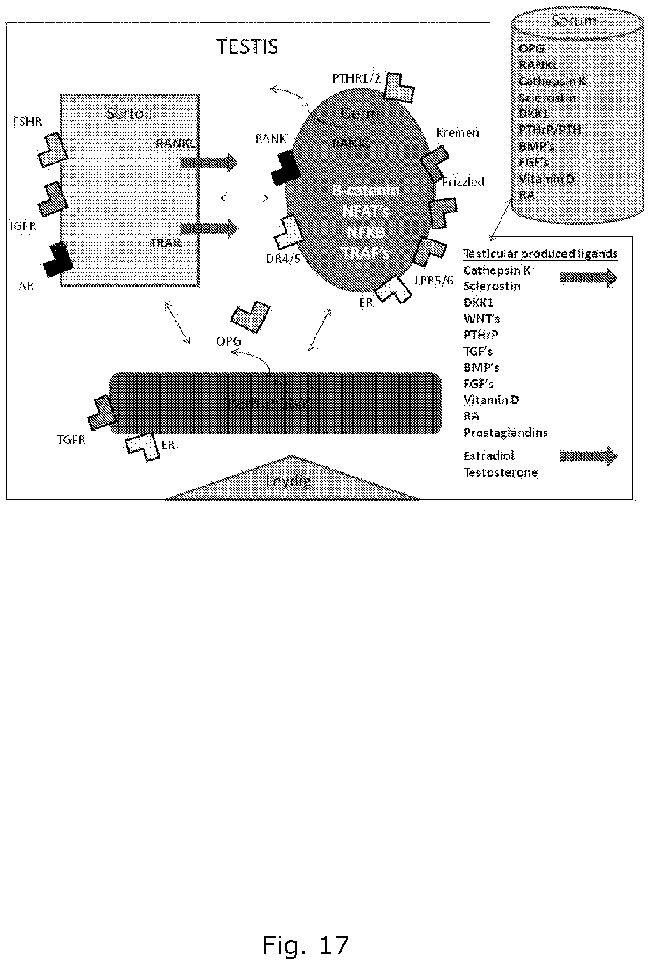

In figure 17, we have listed the most likely regulators and downstream signaling pathways of this paracrine RANKL signaling. We have identified the major regulators and the fact that they are expressed in the testis indicates a function in the gonad. One cannot exclude that it is a combined effect of the local paracrine production of ligands and the systemic contribution which for most of the factors come from the skeleton and will be highlighted in the next example by showing that men with higher serum level of OPG (inhibitor RANKL) also have higher semen concentration.

Discussion

Here we show that several regulators, downstream mediators and interconnected factors to the RANKL, RANK and OPG system are expressed in the testis. This pattern of expression is conserved from mouse to humans for most of the investigated genes, which indicates that this signalling may be involved in spermatogenesis and testis function. It is known that signalling from peritubular and Sertoli cells determines germ cell fate and to our knowledge, this is the first signalling involving peritubular, Sertoli and Germ cells.

The RANKL-system and all the regulators are important for bone homeostasis. The factors involved include the transmembrane RANKL protein in osteoblasts that binds and activates a specific receptor RANK in osteoclasts, which induces differentiation of the immature osteoblasts and results in bone resorption. OPG is an endogenous secreted signalling molecule that binds the ligand domain of RANKL and thus inhibits RANK activation and subsequently osteoclast differentiation and activation, which impairs bone resorption. The American pharmaceutical company Amgen developed a synthetic OPG analog (Denosumab) with a long half-life, and EMEA and FDA approved the drug for the treatment of osteoporosis. Denosumab is now used clinically and has very few side effects since RANKL is considered to be expressed only in immune cells, teeth and breast besides bone. FDA or EMEA did not request a male fertility study before the drug could be used clinically because patients with osteoporosis in general are females and of old age.

We have new data showing that one isoform of RANKL is expressed in the male gonad but not in the female counterpart. This RANKL isoform appears to be expressed in Sertoli cells, while the specific receptor RANK is expressed in spermatogonia, and OPG is expressed in peritubular cells. Expression of these proteins is found in both mice and men, and but the RANKL system are heavily regulated and influenced as shown in the overview figure. All the downstream mediators of RANK signalling are also expressed in the testis from rodents and men, and especially NFKβ is known to be important for the proliferation-apoptotic switch in the male gonad. Rankl signalling may thus be an important regulator of germ cell proliferation. Inhibition of RANKL in the normal testis may mainly be the Sertoli specific form as it is approachable on the proximal site of the blood-testis barrier, while the presumed cytoplasmic form being expressed in the later stages of spermatogenesis probably is inaccessible for circulating Denosumab. The effect of Denosumab cannot be adapted directly from experiments using OPG treatment as Amgen suggested that Denosumab unlike OPG does not block TRAIL, which is important for male germ cell apoptosis but only RANKL. However one cannot exclude that some of the beneficial effect comes from inhibition of TRAIL. The positive effect indicates that the presence of signals upstream and downstream for RANKL in the testis supports that factors such as DKK1, Sclerostin and Cathepsin K that can modified pharmacologically may be new important tools in modifying reproductive function clinically. Especially since all the bone factors are expressed in close proximity with the stem cell pool, whicg means the site of proliferation. The effect is on the spermatogonia and their proliferation determines or is at least the main determinant of total sperm production.

In conclusion, The presence of RANKL, RANK and OPG besides all the classical regulators and downsream mediators in the gonad has never been shown before, and their expression indicates that these bone signals may be involved in regulation of proliferation of germ cells. This implies that regulation of the these bone signals may be clinically relevant as it may lower or increase the production of spermatozoa.

## Example 7

OPG as biomarker e.g. in serum for male fertility or sperm count

In vivo effects of inhibition of testicular RANKL inhibition by infusing human OPG-FC, or Denusomab in humans is the ultimate challenge. However, to get their, we simply measured serum level of OPG and associated the data with the reproductive phenotype to see if systemic serum levels of OPG were associated with sperm or not.

*Materials and methods*

Copenhagen bone gonadal study:

We measured serum OPG by using Immundiagnostic KITS for ELISA. We used all the samples collected at baseline (~300 men).

| | |
|---|---|
| Ages Eligible for Study: | 18 Years and older |
| Genders Eligible for Study: | Male |
| Accepts Healthy Volunteers: | No |

Inclusion Criteria:

- Male with an age > 18 years old

- Referred for male infertility with sperm concentration >= 0.01 million/ml.

Additionally, all men must have either sperm concentration < 20 million/ml or < 50% progressive motile spermatozoa or < 12% morphological normal spermatozoa using strict criteria

Exclusion Criteria:

- Men with chronic diseases (such as diabetes mellitus, Thyroid disease, endocrine disturbances in need of treatment, malignant disease, or diseases known to interfere with VD intake or very sensitive to VD intake (such as inflammatory disease with granuloma: sarcoidoses, tuberculosis, Wegeners, vasculitis, inflammatory bowel disease (Crohn's and colitis ulcerosa etc).

- Men with present or previous malignant disease

- If there is an indication for testis biopsy and it is planned or conducted within the next 6 months

- Serum 25-hydroxy-D3 > 50 nmol/l at the time of inclusion

- Serum Calcium ion > 1,35 mmol/l

- Inhibin-B < 30 pg/ml

- Intake of vitamin D above 15 ug daily

- Allergy towards vitamin D or arachidis oil (peanuts)

- Men with total or partly obstructive oligospermia and men who had vasectomy performed

Criteria for drop out:

- Abrogation of the treatment

- Newly diagnosed endocrine, calcium metabolic disease, parathyroid, thyroid, diabetes or other endocrine disease in need of treatment

- New malignant disease

- Treatment with chemotherapy, immunomodulating therapy, salazopyrin

- Oral or iv treatment with steroid hormones

- Treatment with diuretics, antihypertensive treatment, treatment the heart, calcium channel blockers

- Development of vitamin d intoxication

- If testis biopsy is performed or other surgery in the genital region during the trial

*Results*

OPG was measurable in all investigated men and the assay measured technically acceptable. We noticed that especially men with high OPG and as a result high RANKL inhibition had more sperm. In fact men with serum OPG > 4 pg/ml had a significantly higher sperm concentration than men below 4 pg/ml. If we stratified in tertiles, quintiles, quartiles, The group with highest OPG had significantly more sperm independt of stratification, which indicates a causal relationship although not linear. Instead it makes sense that only high OPG is enough because it is likely that high serum concentrations are needed to exert an effect outside serum especially in the testicular compartment. Moreover, it is evident that only high OPG serum levels results in significantly increased sperm concentration. Serum RANKL is not associated with sperm concentration.

*Discussion/conclusion:*

Serum levels of OPG are positively associated with sperm concentration in infertile men and men with serum OPG >4 or in the highest 20% centil have significant higher sperm concentration compared with infertile men below these levels. This supports the fact that RANKL inhibition stimulates sperm production. Especially testicular RANKL as circulating RANKL was not associated with sperm concentration. This highlights that inhibition of testicular RANKL may be a way to improve semen quality. Either directly with Denusomab or indirect by modifying testicular DKK1, Sclerostin, cathepsin K etc with specific inhibitors or antibodies. Finally, the fact that only men with high OPG have significantly higher sperm concentration indicate that circulating OPG levels can be used to determine which men who will benefit from Denusomab treatment. See also example 8.

**Example 8**

Injection of Denosumab 60 mg sc x 1 into infertile men.

To address the question whether inhibition of RANKL (exemplified by Denosumab) affects human male reproduction, we injected Denosumab 60 mg once into 12 infertile men. All 12 infertile men had impaired semen quality and low sperm counts although the severity varied from mild to severe oligospermia. They had healthy bone evaluated by DXA and normal calcium homeostasis determined by calcium, phosphate, alkaline phosphatase, 25-OHD and PTH. All men delivered 2 semen samples prior to treatment start. They had a blood sample taken for biochemical analysis including serum OPG at baseline. All men were given Denosumab and requested to come in and deliver semen and blood samples at day 5, 20, 40, 80, 120, and 180. Spermatogenesis takes in humans 70 days, so the reason why we asked them to come in early was due to safety (hypocalcemia) and a risk that we would miss an anti-apoptotic effects which theoretically could come earlier (since RANKL inhibition is fast) than a classical proliferation inducing effect that follows the rules of spermatogenesis. There was no significant change in total sperm number, sperm concentration or total number of motile sperm at day 5, 20, and 40. At day 80 there was a significant increase in the number of motile sperm (p=0.05) and the same trend for progressive motile sperm (figure 26A). One man was lost to follow up and one man had high fever.

Interestingly, all the men that had an increase (60%) in sperm number had 200-700% increase in the number of motile sperm. The remaining 40% actually had a decrease in sperm production. This implies that the effect of Denosumab depends on the baseline patient characteristics. We therefore looked into differences in their clinical and biochemical data. A hypoptheis was that men with high RANKL activity and maybe high RANKL/OPG ratio in the testis would benefit the most. This data cannot be revealed without a biopsy and when we measured serum RANKL using the ELISA from immunodiagnostic we found a marked variability in these measurements. However, serum OPG was reliable and actually serum OPG at baseline determined whether or not Denosumab would be beneficial or not (figure 26B). This is important because serum OPG prior to treatment start may assist in selecting men that will benefit from the treatment and thereby avoiding causing a decreased sperm production or no effect in this subgroup of patients.

In sum, the results indicate that higher levels of OPG (such as in blood serum levels) is indicative of a successful treatment. Data indicate that a threshold level in the range 3-5 pmol/l may be relevant, such as 3-4.

**Example 9**

Objective

The aim of the study was to investigate the efficacy and the pharmacodynamic mechanisms of PTH on male reproductive function. We have shown the presence of PTH signalling (ligands and receptors) in the testis. By injecting PTH we will investigate whether testicular and epididymal weight is changed. The testis weight is determined mainly (95%) by the seminiferous tubules, which contains germ and Sertoli cells. Sertoli cells cannot divide after puberty and any increase in testis weight following treatment start will be due to an increase in germ cells as we also showed following OPG infusion in male mice in a previous example.

Justification

Mice were used as the test model because of their suitability in this type of study. The described dosing of test article was used as this will be the intended clinical way of administration. The present mouse model is accepted for the evaluation of treatment with this test article because the receptor/ligands are expressed in the testis of both mice and men and therefore an acceptable model. This study is conducted in accordance with Pipeline Biotech A/S license number 2012-15-2934-00577.
Compound: PTH 1-34 (Forsteo)

Species, strain, specification and supplier

In the study, 30 male C57BL/6J mice supplied by Taconic Europe A/S were used. The animals were 7 week of age at the arrival to the test facility. The test model is an in vivo mice model suited for testing efficacy and pharmacodynamic mechanisms.

| Gr. No. | Mice (=n) | Test article (ID) | Dose | Route | Adm. per week* | Days |
|---------|-----------|-------------------|------|-------|----------------|------|
| 1 | 10 | PTH 1-34 | 60 $\mu$g/kg | s.c. | 5 | 14 |
| 2 | 10 | Anti-sclerostin | 100 mg/kg | i.p. | 1 | 14 |
| 3 | 5 | PTH 1-34 | 60 $\mu$g/kg | s.c. | 5 | 35 |
| 4 | 5 | Vehicle | - | i.p. | 3 | 35 |

Tissue sampling

Kidneys, testicles (+epididymis) and femur bone were dissected free and weighed. Kidney samples: One (1) kidney was snap frozen and 1 was fixed in PFA fixative. Epididymis samples: The epididymis were dissected free from the testis and snap frozen separately. Care was taken not to squeeze the epididymis too much, since the gland contains semen for analysis.
Testis samples after dissection of epididymis: One testis was bisectioned and one half was fixed in PFA fixative and the other part was fixed in bouins fixative. The other testis was snap frozen.

Results

All animals were administered according to the protocol. The animals were not administered at the day of termination. The animals were observed at a daily basis and clinical signs were recorded. No clinical signs were observed at any of the mice in study. The development of the body weight in all groups were similar and developing in a steady rate not different from body weight development in the group treated with PBS. From the development in body weights and the observation it is then concluded that all animals were in good health condition throughout the study.
PTH treatment changed testis weight after both 2 and 5 weeks. The difference was not significant (p=0.16) when using crude values although when indexing the data to the mean of vehicle treated mice and using all data from both 2 and 5 weeks then the data became statistically significant (p<0.05) (figure 19). Thereby, supporting that PTH treatment increases germ cell proliferation and testis weight. Epididymal weight was unchanged after two and five weeks PTH treatment (p=0.56 and 0.95, respectively)

Conclusion

Based on this study it is evident with the line of evidence showing for OPG treatment in a similar mouse model that the increase in testis weight following PTH treatment is a marker for increased germ cell proliferation. PTH is therefore a novel inducer of germ cell proliferation and an inducer of sperm counts. PTH may therefore be valuable treatment in men if you want to increase sperm production.

**Example 10**

Testing the role of ANTI-SCLEROSTIN in the regulation of germ cell proliferation

Objective

The aim of the study was to investigate the efficacy and the pharmacodynamic mechanisms of ANTI-SCLEROSTIN on male reproductive function. We have shown the presence of ANTI-SCLEROSTIN signalling (ligands and receptors) in the testis. By injecting ANTI-SCLEROSTIN we will investigate whether testicular and epididymal weight is changed.

Test article: Anti-sclerostin (antibody)

For further details see also ex 9.

Setup:

| Gr. No. | Mice (=n) | Test article (ID) | Dose | Route | Adm. per week* | Days |
|---|---|---|---|---|---|---|
| 1 | 10 | Anti-sclerostin | 100 mg/kg | i.p. | 1 | 14 |
| 2 | 10 | Vehicle | - | i.p. | 3 | 14 |
| 3 | 5 | Anti-sclerostin | 100 mg/kg | i.p. | 1 | 35 |
| 4 | 5 | Vehicle | - | i.p. | 3 | 35 |
| *) 1 time/week was Mon. 2 times/week were Mon and Thu. Three (3) times/week were Mon, Wed and Fri. 5 times/week weree Mon-Fri. | | | | | | |

Results

ANTI-SCLEROSTIN treatment changed testis weight after both 2 and 5 weeks. The difference was not significant (p=0.09) when using crude values although when indexing the data to the mean of vehicle treated mice and using all data from both 2 and 5 weeks then the data became statistically significant (p<0.05) (figure 20). Thereby, supporting that ANTI-SCLEROSTIN treatment reduces germ cell proliferation and testis weight. Epididymal weight also changed after five weeks ANTI-SCLEROSTIN treatment (p=0.10). Interestingly femur weight also increased significantly indicating that the antibody worked as expected.

Conclusion

Based on this study it is evident with the line of evidence showed previously for OPG treatment in a similar mouse model that the decrease in testis weight following ANTI-SCLEROSTIN treatment is a marker for lower germ cell proliferation. ANTI-SCLEROSTIN is therefore a novel repressor of germ cell proliferation and of sperm counts. ANTI-SCLEROSTIN may therefore be valuable treatment in men who do not wish to have any sperm counts for instance as anti-conception or in the treatment of testis cancer.

**Example 11**

Testing the role of CHOLECALCIFEROL in the regulation of germ cell proliferation

Objective

The aim of the study was to investigate the efficacy and the pharmacodynamic mechanisms of CHOLECALCIFEROL on male reproductive function. Cholecalciferol is inactive vitamin D and has to be activated before it can bind and activate the vitamin d receptor. We have shown the presence of CHOLECALCIFEROL signalling (ligands and receptors) in the testis. By injecting CHOLECALCIFEROL we will investigate whether testicular and epididymal weight is changed.

Test article: Cholecalciferol (D-draber). Amount of drug substance per unit; 80 IU per drop. Chow will be prepared to contain 150 IU per 6.1 g chow equal to a dose of 5000 IU/kg BW/day.

For further details see also ex 9.

Experimental design

| Gr. No. | Mice (=n) | Test article (ID) | Dose | Route | Adm. per week* | Days |
|---|---|---|---|---|---|---|
| 1 | 10 | Cholecalciferol | 5000 IU/kg/day | in the diet | daily | 14 |

(continued)

| Gr. No. | Mice (=n) | Test article (ID) | Dose | Route | Adm. per week* | Days |
|---|---|---|---|---|---|---|
| 2 | 10 | Vehicle | - | i.p. | 3 | 14 |
| *) 1 time/week was Mon. 2 times/week were Mon and Thu. Three (3) times/week were Mon, Wed and Fri. 5 times/week weree Mon-Fri. | | | | | | |

Results

All animals were administered according to the protocol. The animals were not administered at the day of termination. The animals were observed at a daily basis and clinical signs were recorded. No clinical signs were observed at any of the mice in study. The development of the body weight in all groups was similar and developing in a steady rate not different from body weight development in the group treated with PBS. From the development in body weights and the observation it is then concluded that all animals were in good health condition throughout the study.

CHOLECALCIFEROL treatment changed testis weight after both 2 weeks. The difference was not significant (p=0.09). Although supporting that CHOLECALCIFEROL treatment increases germ cell proliferation and testis weight. Epididymal weight also changed after two weeks CHOLECALCIFEROL treatment (p=0.22) (figure 21).

Conclusion

Based on this study it is evident with the line of evidence showing for OPG treatment in a similar mouse model that the increase in testis weight following CHOLECALCIFEROL treatment is a marker for increased germ cell proliferation CHOLECALCIFEROL is therefore a novel inducer of germ cell proliferation and an inducer of sperm counts. CHOLECALCIFEROL may therefore be valuable treatment in men if you want to increase sperm production either alone or in combination with Denosumab.

**Example 12**

Testing the role of ETALPHA in the regulation of germ cell proliferation

Objective

The aim of the study was to investigate the efficacy and the pharmacodynamic mechanisms of ETALPHA on male reproductive function. Etalpha is 1-alphahydroxylated vitamin D and has to be activated by the 25-hydroxylase before it can bind and activate the vitamin d receptor. We have shown the presence of ETALPHA signalling (metabolizing enzymes and receptors) in the testis. By injecting ETALPHA we will investigate whether testicular and epididymal weight is changed.

Test article: Etalpha

For further details see also ex 9.

Experimental design

| Gr. No. | Mice (=n) | Test article (ID) | Dose | Route | Adm. per week* | Days |
|---|---|---|---|---|---|---|
| 1 | 5 | Etalpha | 0.05 $\mu$g/mice | i.p | 3 x weekly | 14 |
| 2 | 5 | Vehicle | - | i.p. | 3 x weekly | 14 |
| *) 1 time/week was Mon. 2 times/week were Mon and Thu. Three (3) times/week were Mon, Wed and Fri. 5 times/week weree Mon-Fri. | | | | | | |

Results

All animals were administered according to the protocol. The animals were not administered at the day of termination. The animals were observed at a daily basis and decreases significantly in body weight. Therefore, they were terminated already at day 9. ETALPHA treatment changed testis weight after 2 weeks. The difference was significant (figure 22). Supporting that ETALPHA treatment increases germ cell proliferation and testis weight. Epididymal weight did not changed after two weeks ETALPHA treatment.

Conclusion

Based on this study it is evident with the line of evidence showing for OPG treatment in a similar mouse model that the increase in testis weight following ETALPHA treatment is a marker for increased germ cell proliferation ETALPHA is therefore a novel inducer of germ cell proliferation and an inducer of sperm counts. ETALPHA may therefore be valuable treatment in men if you want to increase sperm production either alone or in combination with Denosumab.

**Example 13**

Testing the role of CINACALCET in the regulation of germ cell proliferation

Objective

The aim of the study was to investigate the efficacy and the pharmacodynamic mechanisms of CINACALCET on male reproductive function. Cinacalcet is an agonist for Calcium sensing receptor. We have data showing the presence of CaSR signalling in the testis. By injecting CINACALCET we will investigate whether testicular and epididymal weight is changed.
Test article: Cinacalcet
For further details see also ex 9.

Experimental design

| Gr. No. | Mice (=n) | Test article (ID) | Dose | Route | Adm. per week* | Days |
|---------|-----------|-------------------|------|-------|----------------|------|
| 1 | 5 | Cinacalcet | 100 mg/kg | p.o. | 5 x weekly | 14 |
| 2 | 5 | Vehicle | - | i.p. | 3 x weekly | 14 |
| *) 1 time/week was Mon. 2 times/week were Mon and Thu. Three (3) times/week were Mon, Wed and Fri. 5 times/week weree Mon-Fri. | | | | | | |

Results

CINACALCET treatment changed testis weight after 2 weeks. The difference was significant. Supporting that CINACALCET treatment increases germ cell proliferation and testis weight (figure 23). Epididymal weight did not change significantly after two weeks CINACALCET treatment.

Conclusion

Based on this study it is evident with the line of evidence showing for OPG treatment in a similar mouse model that the increase in testis weight following CINACALCET treatment is a marker for increased germ cell proliferation CINACALCET is therefore a novel inducer of germ cell proliferation and an inducer of sperm counts. CINACALCET may therefore be valuable treatment in men if you want to increase sperm production either alone or in combination with e.g. Denosumab.

**Example 14**

Testing the role of FGF23 in the regulation of germ cell proliferation

Objective

The aim of the study was to investigate the efficacy and the pharmacodynamic mechanisms of FGF23 on male reproductive function. Fgf23 binds to a rececptor klotho. We have data showing the presence of Klotho CaSR signalling in the testis. By injecting FGF23 we will investigate whether testicular and epididymal weight is changed.
Test article: Fgf23

For further details see also ex 9.

Experimental design

| Gr. No. | Mice (=n) | Test article (ID) | Dose | Route | Adm. per week* | Days |
|---------|-----------|-------------------|------|-------|----------------|------|
| 1 | 5 | FGF23 | 50 μg/kg | i.p | 3 x weekly | 14 |
| 2 | 5 | Vehicle | - | i.p. | 3 x weekly | 14 |
| *) 1 time/week was Mon. 2 times/week were Mon and Thu. Three (3) times/week were Mon, Wed and Fri. 5 times/week weree Mon-Fri. | | | | | | |

Results

FGF23 treatment changed testis weight after 2 weeks. The difference was significant (figure 24). Supporting that FGF23 treatment increases germ cell proliferation and testis weight. Epididymal weight did not change significantly after two weeks FGF23 treatment.

Conclusion

Based on this study it is evident with the line of evidence showing for OPG treatment in a similar mouse model that the increase in testis weight following FGF23 treatment is a marker for increased germ cell proliferation. FGF23 is therefore a novel inducer of germ cell proliferation and an inducer of sperm counts. FGF23 may therefore be valuable treatment in men if you want to increase sperm production either alone or in combination with e.g. Denosumab.

**Example 15**

Testing the role of bisphosphonates in the regulation of germ cell proliferation

Objective

The aim of the study was to investigate the efficacy and the pharmacodynamic mechanisms of BISPHOSPHONATES (ALENDRONATE) on male reproductive function. Bisphosphonates (alendronate) increases bone formation. We have data showing that several bone factors are present in the testis and by injecting BISPHOSPHONATES (ALENDRONATE). We will investigate whether testicular and epididymal weight is changed.
Test article: Bisphosphonates (alendronate)

For further details see also ex 9.

Experimental design

| Gr. No. | Mice (=n) | Test article (ID) | Dose | Route | Adm. per week* | Days |
|---------|-----------|-------------------|------|-------|----------------|------|
| 1 | 5 | Alendronate | 2.0 mg/kg | p.o. | 5 x weekly | 14 |
| 2 | 5 | Vehicle | - | i.p. | 3 x weekly | 14 |
| *) 1 time/week was Mon. 2 times/week were Mon and Thu. Three (3) times/week were Mon, Wed and Fri. 5 times/week were Mon-Fri. | | | | | | |

Results

BISPHOSPHONATES (ALENDRONATE) treatment changed testis weight after 2 weeks. The difference was significant (figure 25). Supporting that BISPHOSPHONATES (ALENDRONATE) treatment increases germ cell proliferation and testis weight. Epididymal weight did not change significantly after two weeks BISPHOSPHONATES (ALENDRONATE) treatment.

Conclusion

Based on this study it is evident with the line of evidence showing for OPG treatment in a similar mouse model that the increase in testis weight following BISPHOSPHONATES (ALENDRONATE) treatment is a marker for increased germ cell proliferation BISPHOSPHONATES (ALENDRONATE) is therefore a novel inducer of germ cell proliferation and an inducer of sperm counts. BISPHOSPHONATES (ALENDRONATE) may therefore be valuable treatment in men if you want to increase sperm production either alone or in combination with Denosumab.

**Claims**

1. A method for determining a likely effect of a treatment to improve male fertility, the method comprising

   • determining the level of OPG in a blood serum sample, which has been obtained from a male subject;
   • comparing said determined OPG level to a reference level; and
   • determining that said subject will

      ◦ likely not benefit from treatment with an antagonist or inhibitor of RANKL, if said level is lower than said reference level, or
      ◦ likely benefit from treatment with an antagonist or inhibitor of RANKL, if said level is equal to higher than said reference level;

   wherein said reference level is above 3 pmol/l OPG in blood serum.

2. The method according to claim 1, wherein said determination of the protein level of OPG is performed using a method selected from the group consisting of immunohistochemistry, immunocytochemistry, FACS, ImageStream, Western Blotting, qPCR, RT-PCR, qRT-PCR, ELISA, Luminex, Multiplex, Immunoblotting, TRF-assays, immunochromatographic lateral flow assays, Enzyme Multiplied Immunoassay Techniques, RAST test, Radioimmunoassays, immunofluorescence and immunological dry stick assays.

3. The method according to claim 2, wherein said determination is performed by immunocytochemistry.

4. The method according to claim 2, wherein said determination is performed by ELISA.

5. The method according to any of the preceding claims, wherein said reference level is in the range in the range 3 to 5 pmol/l OPG, such as in the range 3 to 4 pmol/l OPG.

6. The method according to any of the preceding claims, wherein said inhibitor of RANKL is Denosumab.

**Patentansprüche**

1. Verfahren zum Bestimmen eines wahrscheinlichen Effekts einer Behandlung zur Verbesserung männlicher Unfruchtbarkeit, wobei das Verfahren Folgendes umfasst:

   • Ermitteln des OPG-Spiegels in einer Blutserumprobe, die von einem männlichen Individuum gewonnen wurde;
   • Vergleichen des ermittelten OPG-Spiegels mit einem Referenzspiegel; und
   • Feststellen, dass das Individuum

      ◦ wahrscheinlich nicht von der Behandlung mit einem RANKL-Antagonisten oder -Inhibitor profitieren wird, wenn der Spiegel niedriger als der Referenzspiegel ist, oder

∘ wahrscheinlich von der Behandlung mit einem RANKL-Antagonisten oder - Inhibitor profitieren wird, wenn der Spiegel gleich oder höher als der Referenzspiegel ist;

wobei der Referenzspiegel über 3 pmol/l OPG im Blutserum beträgt.

2. Verfahren nach Anspruch 1, wobei das Ermitteln des OPG-Proteinspiegels unter Verwendung eines Verfahrens durchgeführt wird, das aus der Gruppe ausgewählt ist, die aus Immunhistochemie, Immunzytochemie, FACS, Image-Stream, Western-Blotting, qPCR, RT-PCR, qRT-PCR, ELISA, Luminex, Multiplex, Immun-Blotting, TRF-Assays, immunchromatographischen Lateral Flow Assays, Enzyme-Multiplied Immunoassay Techniques, RAST-Test, Radioimmunassays, Immunfluoreszenz- und immunologischen Assays mit Trockenteststreifen besteht.

3. Verfahren nach Anspruch 2, wobei das Ermitteln mit Immunzytochemie durchgeführt wird.

4. Verfahren nach Anspruch 2, wobei das Ermitteln mit ELISA durchgeführt wird.

5. Verfahren nach einem der vorstehenden Ansprüche, wobei der Referenzspiegel im Bereich von 3 bis 5 pmol/l OPG, wie etwa im Bereich von 3 bis 4 pmol/l OPG liegt.

6. Verfahren nach einem der vorstehenden Ansprüche, wobei der RANKL-Inhibitor Denosumab ist.

## Revendications

1. Procédé destiné à déterminer un effet probable d'un traitement pour améliorer la fertilité masculine, le procédé comprenant

• la détermination du niveau d'OPG dans un échantillon de sérum sanguin, qui a été obtenu d'un sujet masculin ;
• la comparaison dudit niveau d'OPG déterminé avec un niveau de référence ; et
• la détermination que ledit sujet est

∘ susceptible de ne pas bénéficier d'un traitement avec un antagoniste ou un inhibiteur de RANKL, si ledit niveau est inférieur audit niveau de référence, ou
∘ susceptible de bénéficier d'un traitement avec un antagoniste ou un inhibiteur de RANKL, si ledit niveau est supérieur ou égal audit niveau de référence ;

dans lequel ledit niveau de référence est au-dessus de 3 pmol/l d'OPG dans le sérum sanguin.

2. Procédé selon la revendication 1, dans lequel ladite détermination du niveau protéique d'OPG est réalisée en utilisant un procédé sélectionné parmi le groupe constitué par l'immunohistochimie, l'immunocytochimie, le FACS, ImageStream, le buvardage Western, qPCR, RT-PCR, qRT-PCR, ELISA, Luminex, Multiplex, l'immunobuvardage, les dosages de TRF, les dosages immunochromatographiques à flux latéral, les techniques d'immunodosage enzymatique multiplié, le test RAST, les radioimmunodosages, les dosages d'immunofluorescence et à bâtonnets secs immunologiques.

3. Procédé selon la revendication 2, dans lequel ladite détermination est réalisée par immunocytochimie.

4. Procédé selon la revendication 2, dans lequel ladite détermination est réalisée par ELISA.

5. Procédé selon l'une quelconque des revendications précédentes, dans lequel ledit niveau de référence est dans la plage de 3 à 5 pmol/l OPG, tel que dans la plage de 3 à 4 pmol/l OPG.

6. Procédé selon l'une quelconque des revendications précédentes, dans lequel ledit inhibiteur de RANKL est le Dénosumab.

| NR | ID |
|---|---|
| 1 | Seminoma |
| 2 | Seminoma |
| 3 | Seminoma |
| 4 | Teratoma |
| 5 | Embryonal carcinoma |
| 6 | Embryonal carcinoma |
| 7 | Mixed tumor |
| 8 | CIS |
| 9 | CIS |
| 10 | CIS |
| 11 | Normal Testis |
| 12 | Normal Testis |
| 13 | Sertoli cell only |
| 14 | NT2 cells DAY 0 |
| 15 | NT2 cells DAY 15 |
| 16 | Cell line 2102EP |
| 17 | Cell line TCAM 2 |
| 18 | Cell line JKT |
| 19 | Epididymis |
| 20 | Seminal vesicle |
| 21 | Ovary |
| 22 | Kidney |
| 23 | H2O |

Fig. 1

| NR | ID |
|----|--------|
| 1 | Vdr WT |
| 2 | Vdr WT |
| 3 | Vdr WT |
| 4 | Vdr WT |
| 5 | Vdr WT |
| 6 | Vdr KO |
| 7 | Vdr KO |
| 8 | Vdr KO |
| 9 | Vdr KO |
| 10 | Vdr KO |

Fig. 2A

Fig. 2B

Fig. 3

Fig. 4

Fig. 5

Fig. 6

Fig. 7

Fig. 8

Fig. 9

Fig. 10

Fig. 11

| NR | ID | |
|----|------|---|
| 0 | | |
| 1 | 5703 V I | SEMINOMA |
| 2 | 5977 H I | SEMINOMA |
| 3 | 6246 H I | SEMINOMA |
| 4 | 5117 H I | TERATOMA |
| 5 | 5995 V I | EMBRYONAL CARCINOMA (EC) |
| 6 | 6797 V I | EC |
| 7 | 5853 V I | EC/YST/CH |
| 8 | 5514 V II | CARCINOMA IN SITU (CIS) 50-80 |
| 9 | 6220 V | CIS 95 |
| 10 | 6204 I | CIS 95 |
| 11 | NT Clontech | Normal testis |
| 12 | NT BioChain | Normal testis |
| 13 | 6118 H II | Sertoli cell only |
| 14 | NT2 DAY 0 | NT2 cell DAY 0 |
| 15 | NT2 DAY 15 | NT2 cell DAY 15 |
| 16 | 2102EP | 2102EP cell |
| 17 | TCAM 2 | TCAM 2 cell |
| 18 | JKT | Prostata |
| 19 | Epi | Epididymis |
| 20 | VS1 | Seminal Vesicle |
| 21 | Købe | OVARY |
| 22 | Coltech | KIDNEY |
| 23 | H2O | H2O |

Fig. 12, continues

**1. Primers: hDKK1**

0 1 2 3 4 5 6 7 8 9 10 11 12 13 14 15 16 17 18 19 20 21 22 23

**2. Primers: hLRP5**

0 1 2 3 4 5 6 7 8 9 10 11 12 13 14 15 16 17 18 19 20 21 22 23

**3. Primers: hLRP6**

0 1 2 3 4 5 6 7 8 9 10 11 12 13 14 15 16 17 18 19 20 21 22 23

**4. Primers: hCathepsin K**

0 1 2 3 4 5 6 7 8 9 10 11 12 13 14 15 16 17 18 19 20 21 22 23

# Fig. 12, continued

1. mNFATc1

0  1  2  3  4  5  6  7  8  9  10  11 12 13  14

2. mDKK1

0  1  2  3  4  5  6  7  8  9  10  11 12 13  14

3. mLRP4

0  1  2  3  4  5  6  7  8  9  10  11 12 13  14

4. mLRP5

0  1  2  3  4  5  6  7  8  9  10  11 12 13  14

5. mLRP6

0  1  2  3  4  5  6  7  8  9  10  11 12 13  14

6. mkremen 1

0  1  2  3  4  5  6  7  8  9  10  11 12 13  14

7. mkremen 2

0  1  2  3  4  5  6  7  8  9  10  11 12 13  14

Fig. 13

**A**

**B**

**C**

Fig. 14

**A**

**B**

|  | NT | CIS | SEM | EC |
|---|---|---|---|---|
| Cathepsin K | | | | |
| B-actin | | | | |

Fig. 15

Fig. 16

Fig. 17

**Sperm concentration ($10^6$)**

Fig. 18

Fig. 19

**A**

**B**

**C**

Fig. 20

**A**

**B**

Fig. 21

Fig. 22

Fig. 23

**A**

Fig. 24

Fig. 25

**A**

**B**

Fig. 26

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013067639 A1
- WO 8801649 A
- US 4946778 A
- US 5260203 A
- US 4816567 A
- US 4411993 A
- US 4703004 A

### Non-patent literature cited in the description

- *Nature Medicine,* 2005, vol. 11, 880-885
- **KOHLER et al.** *Nature,* 1975, vol. 256, 495
- *Rognes,* 2001
- *CHEMICAL ABSTRACTS,* 909395-70-6
- **ASAGIRI M et al.** Cathepsin K-dependent toll-like receptor 9 signaling revealed in experimental arthritis. *Science,* 2008, vol. 319 (5863), 624-627
- **HUSSEIN, H. et al.** Pharmacokinetics and bone resorption evaluation of a novel Cathepsin K inhibitor (VEL-0230) in healthy adult horses. *Journal of veterinary pharmacology and therapeutics,* 2014
- *CHEMICAL ABSTRACTS,* 59865-13-3
- *CHEMICAL ABSTRACTS,* 249537-73-3
- **LACEY et al.** *Nature Reviews,* 2012, vol. 11, 401-419
- **KOHLER et al.** *Nature,* 1975, vol. 256, 495-497
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **HARLOW ; LANE.** Antibodies: A Laboratory Manual. Cold Spring Harbor Laboratory, 1988
- **GREEN, LL.** *J. Immunol. Methods,* 1999, vol. 231, 11-25
- **HARLOW et al.** Antibodies: A Laboratory Manual. Cold Spring Harbor Laboratory Press, 1988
- Current Protocols in Immunology. Greene Publishing Assoc, 1992
- **MULLER.** *Meth. Enzymol.,* 1983, vol. 92, 589-601
- **CABILLY et al.** *Proc. Natl. Acad. Sci. USA,* 1984, vol. 81, 3273-3277
- **MORRISON et al.** *Proc. Natl. Acad. Sci. USA,* 1984, vol. 81, 6851-6855
- **BOULIANNE et al.** *Nature,* 1984, vol. 312, 643-646
- **NEUBERGER et al.** *Nature,* 1985, vol. 314, 268-270
- **LIU et al.** *Proc. Natl. Acad. Sci. USA,* 1987, vol. 84, 3439-3443
- **MORRISON et al.** *Proc. Natl. Acad. Sci.,* 1985, vol. 81, 6851-6855
- Current Protocols in Molecular Biology. Wiley Interscience, 1993
- **PARDOLL ; BECKERLEG.** *Immunity,* 1995, vol. 3, 165
- **RAZ et al.** *Proc. Natl. Acad. Sci. USA,* 1994, vol. 91, 9519
- **WANG et al.** *Proc. Natl. Acad. Sci. USA,* 1993, vol. 90, 4156
- **ENGVALL et al.** *Immunochem,* 1971, vol. 8, 871
- **BECKMAN et al.** *J. Immunol.,* 1990, vol. 144, 4212
- **BLOMBERG JENSEN M et al.** Characterization of the testicular, epididymal and endocrine phenotypes in the Leuven Vdr-deficient mouse model: targeting estrogen signalling. *Mol Cell Endocrinol,* 11 July 2013, vol. 377 (1-2), 93-102